(19) Europäisches Patentamt
European Patent Office
Office européen des brevets

(11) Publication number: **0 054 233**
**B1**

(12) # EUROPEAN PATENT SPECIFICATION

(45) Date of publication of patent specification: **24.09.86**

(21) Application number: **81110131.0**

(22) Date of filing: **04.12.81**

(51) Int. Cl.⁴: **C 07 D 409/06,**
C 07 D 409/14,
C 07 D 405/06,
C 07 D 417/14,
C 07 D 405/14,
C 07 D 413/12, A 61 K 31/38,
A 61 K 31/335,
A 61 K 31/395, A 01 N 43/50,
A 01 N 43/653 // C07D409/12,
C07D335/06

(54) **Novel benzo(b)thiophenes, benzo(b)furans, thiochromans and chromans, processes for their preparation and antimicrobial compositions containing them.**

(30) Priority: **12.12.80 US 215948**

(43) Date of publication of application:
**23.06.82 Bulletin 82/25**

(45) Publication of the grant of the patent:
**24.09.86 Bulletin 86/39**

(84) Designated Contracting States:
**AT BE CH DE FR GB IT LI NL SE**

(56) References cited:
**GB-A-1 445 707**

**A. BURGER; Medicinal Chemistry, Part I, WILEY INTERSCIENCE; NEW YORK, 72-80 (1970)**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Rane, Dinanath Fakir**
**21 Briarwood Ct**
**Emerson New Jersey 07630 (US)**
Inventor: **Wright, John Jessen**
**4 Todd Terrace**
**Cedar Grove New Jersey 07009 (US)**
Inventor: **Pike, Russell Edwin**
**31 Florence Street (IFDI)**
**Stanhope New Jersey 07874 (US)**

(74) Representative: **Ritter, Stephen David et al**
**Mathys & Squire 10 Fleet Street**
**London EC4Y 1AY (GB)**

**EP 0 054 233 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel benzo[b]thiophenes, benzo[b]furans, thiochromans and chromans having antimicrobial activity, to processes for their preparation and to antimicrobial compositions containing them.

British Patent Specification No. 1,445,707 describes 1-benzyloxy-2-(1-imidazolyl)-indones and -tetralines having antimicrobial properties.

According to the invention we provide compounds of the formula

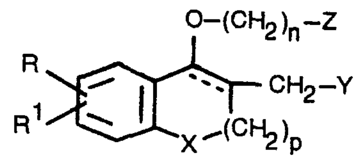

I

wherein

$n$ is 0 or an integer from 1 to 4;

$p$ is 0 or 1;

R and $R^1$ are independently hydrogen or halogen atoms, or lower alkyl, haloalower alkyl, nitro or $NR^2R^3$ groups, wherein $R^2$ and $R^3$ are independently hydrogen atoms or lower alkyl groups; or R and $R^1$ together in adjacent positions complete a fused saturated or unsaturated carbo-cyclic ring that may contain 5 to 7 carbon atoms;

X is an oxygen or sulfur atom or a sulphinyl or sulphonyl group;

Y is an imidazolyl or 1,2,4-triazolyl group, either of which may be substituted by a lower alkyl group or by a phenyl, halophenyl or lower alkyl phenyl group;

Z is an alkyl group having 1 to 10 carbon atoms, an alkenyl or alkynyl group having 3 to 10 carbon atoms, a cycloalkyl group having 3 to 6 carbon atoms or a heterocyclic aromatic group selected from imidazolyl, thiazolyl, isothiazolyl, pyrazolyl, pyridyl, thienyl, thiadiazolyl, quinolyl and furanyl, said heterocyclic aromatic groups being unsubstituted or substituted by at least one substituent selected from halogen atoms and lower alkyl, lower alkoxy, lower alkylidenedioxy, phenyl and N-(N'-lower alkanoylpiperazinyl) groups, provided that, when $n$ is 0, Z is not 1-alkynyl; and, when $n$ is 1 to 4, Z can also be an alkoxy or alkylthio group having 1 to 10 carbon atoms, or a phenyl or phenoxy group, or a phenyl or phenoxy group having substituents selected from halogen atoms and lower alkyl, lower alkoxy, lower alkylidenedioxy and N-(N'-lower alkanoylpiperazinyl) groups; where the term "lower" indicates groups having 1 to 6 carbon atoms; and the broken line indicates a single or double bond, with the proviso that it is a double bond only when $p$ is 0 and X is a sulfur atom; and the acid addition salts thereof.

The imidazolyl and 1,2,4-triazolyl groups are preferably attached through the nitrogen atom at their 1-positions.

"Halogen" refers to fluorine, chlorine, bromine and iodine. The term "lower" indicates groups having 1 to 6, preferably 1 to 4 carbon atoms, e.g., "lower alkyl" preferably refers to hydrocarbon chains of 1 to 4 carbon atoms, for example methyl, ethyl, *n*-propyl, iso-propyl, *n*-butyl or *t*-butyl groups. Alkyl, alkoxy, alkylthio, alkenyl and alkynyl groups in Z may be straight or branched and have up to 10 carbon atoms. These include the aforementioned lower alkyl groups and also *n*-pentyl, *n*-hexyl, 2-ethylpentyl, and *t*-decyl groups; methoxy, ethoxy, propoxy and decyloxy groups; methylthio, ethylthio and decylthio groups; 1-propenyl, 2-propenyl, 2-pentenyl, 3-hexenyl, 5-octenyl and 2-decenyl groups; and propynyl, 2-butynyl, 2-pentynyl and 5-decynyl groups. It should be noted that the alkenyl and alkynyl groups have *3* to 10 carbon atoms. Cycloalkyl groups in Z have 3 to 6 carbon atoms and include cyclopropyl, cyclobutyl, cyclopentyl and cyclohexyl groups.

Substituted phenyl groups in Z include in particular mono-, di- and tri-halophenyl and mono-, di- and tri-lower alkylphenyl groups, e.g. 4-chlorophenyl, 2,4-dichlorophenyl, 2,6-dichlorophenyl, 2,4,6-trichlorophenyl, tolyl, xylyl and mesityl groups.

The alkanoyl groups included in the term "N-(N'-alkanoylpiperazinyl)" may be straight or branched and have up to 6 carbon atoms, for example formyl, acetyl, or butyryl.

Aromatic heterocyclic groups included in Z may be unsubstituted or substituted by at least one substituent selected from halogen atoms and lower alkyl groups, especially 2-thienyl, 3-thienyl, 2-chloro-3-thienyl, 5-chloro-2-thienyl, 2,5-dichloro-3-thienyl, pyridyl, quinolyl, thiazolyl, and furanyl groups.

The compounds of the formula I wherein the broken line indicates a single bond can exist as *cis*- and *trans*-isomers (*cis*-2,3 and *trans*-2,3 when $p$ is 0, and *cis*-3,4 and *trans*-3,4 when $p$ is 1), each of which is a racemate and can be resolved into its optically active enantiomers e.g. by salt formation with an optically active acid and fractional crystallisation. The present invention embraces all such forms; the *cis*-forms are normally preferred to the *trans*-forms on account of their superior antimicrobial properties.

In a preferred group of compounds of the formula I, X, Y, $n$ and $p$ are as defined above,

R and $R^1$ are independently hydrogen or halogen atoms, or lower alkyl, halolower alkyl, nitro or $NR^2R^3$

groups, wherein $R^2$ and $R^3$ are independently hydrogen atoms or lower alkyl groups,

Z is an alkyl, alkenyl, alkynyl or cycloalkyl group or a heterocyclic aromatic group defined above which may have at least one substituent selected from halogen atoms and lower alkyl groups; provided that, when *n* is 0, Z is not 1-alkynyl; and, when *n* is 1 to 4, Z can also be an alkoxy, alkylthio, phenyl, aryloxy or arylthio group, or a phenyl group having substituents selected from halogen atoms and lower alkyl and N-(N'-lower alkanoylpiperazinyl) groups, the term "lower indicates groups having 1 to 4 carbon atoms, and the broken line indicates a single bond.

More especially, in these compounds X is a sulfur atom and *p* is 0; Y is preferably a 1-imidazolyl group and Z is preferably a heterocyclic aromatic group; another group of such preferred compounds can be represented by the formula

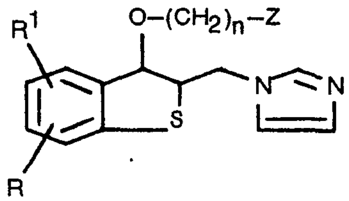

II

wherein R, $R^1$, Z and *n* are as defined in the preceding sentence.

In the compounds of formula II, Z is preferably a phenyl or heterocyclic aromatic group which may be substitued by a halogen atom or a lower alkyl group, e.g. a thienyl or 2-chlorothienyl group. In another group of preferred compounds of the formula II *n* is 1, Z is chlorophenyl or dichlorophenyl, and R and $R^1$ are hydrogen or halogen atoms.

Particularly preferred compounds of formula I (on account of their especially favourable antimicrobial properties) include

*cis*-6-chloro-3-(2'-chloro-3'-thenyloxy)-2,3-dihydro-2-(1''-imidazolylmethyl)benzo[b]thiophene,
*cis*-6-chloro-3-(3'-chloro-3'-thenyloxy)-2,3-dihydro-2-(1''-imidazolylmethyl)benzo[b]thiophene,
*cis*-6-fluoro-3-(2'-chloro-3'-thenyloxy)-2,3-dihydro-2-(1''-imidazolylmethyl)benzo[b]thiophene, and
*cis*-5-fluoro-3-(2'-chloro-3'-thenyloxy)-2,3-dihydro-2-(1''-imidazolylmethyl)benzo[b]thiophene.

Other preferred compounds of formula I include

*cis*-6-chloro-3-(2'-chloro-3'-thenyloxy)-2,3-dihydro-2-(1''-imidazolylmethyl)benzo[b]thiophene-1-oxide,
*cis*-5-chloro-3-(2'-chloro-3'-thenyloxy)-2,3-dihydro-2-(1''-imidazolylmethyl)benzo[b]thiophene-1-oxide,
6-chloro-3-(2'-chloro-3'-thenyloxy)-2-(1''-imidazolylmethyl)benzo[b]thiophene,
5-chloro-3-(2'-chloro-3'-thenyloxy)-2-(1''-imidazolylmethyl)benzo[b]thiophene,
*cis*-6-chloro-3-(6'-chloro-3',4'-methylenedioxybenzyloxy)-2,3-dihydro-2-(1''-imidazolylmethyl)-benzo[b]thiophene,
*cis*-6-chloro-3-(2'-chloro-6'-fluorobenzyloxy)-2,3-dihydro-2-(1''-imidazolylmethyl)benzo[b]thiophene,
*cis*-6-chloro-3-[(5'-chloro-3'-methyl-1'-phenyl-pyrazol-4'-yl)methoxy]-2,3-dihydro--2-(1''-imidazolylmethyl)benzo[b]thiophene,
*cis*-6-chloro--3-(2'-chlorobenzo[b]thienyl-3'-methoxy)-2,3-dihydro-2-(1''-imidazolylmethyl)-benzo[b]thiophene,
*cis*--3-(2'-chloro-3'-thenyloxy)-2,3,5,6,7,8-hexahydro-2-(1''-imidazolylmethyl)naphtho[2,3-b]thiophene
*cis*-3-(2'-chloro-3'-thenyloxy)-2,3-dihydro-2-(1''-imidazolylmethyl)naphtho[1,2-b]thiophene,
*cis*-3-(2'-chloro-3'-thenyloxy)-2,3,4,5,6,7-hexahydro-2-(1''-imidazolylmethyl)naphtho[3,4-b]thiophene,
*cis*-6-chloro-3-allyloxy-2,3-dihydro-2-(1'-imidazolylmethyl)benzo[b]thiophene,
*cis*-6-chloro-3-(quinolyl-2'-methoxy)-2,3-dihydro-2-(1''-imidazolylmethyl)benzo[b]thiophene,
*cis*-6-chloro-3-(4'-thiazolylmethoxy)-2,3-dihydro-2-(1''-imidazolylmethyl)benzo[b]thiophene,
*cis*-6-chloro-2,3-dihydro-3-[4'-(5'-methyl-3'-phenylisoxazolyl)methoxy]-2-(1''-imidazolylmethyl)-benzo[b]thiophene,
*cis*-6-chloro-3-{8'-chloro-1',3'-dioxolo[4',5'-g]quinolin-7'-ylmethoxy}-2,3-dihydro-2-(1''-imidazolylmethyl)benzo[b]thiophene,
*cis*-6-chloro-3-(2',5'-dichloro-3'-thenyloxy)-2,3-dihydro-2-(1''-imidazolylmethyl)benzo[b]thiophene,
*cis*-6-chloro-3-(4'-chlorobenzyloxy)-2,3-dihydro-2-(1''-imidazolylmethyl)benzo[b]thiophene.
*cis*-6-chloro-3-(2',4'-dichlorobenzyloxy)-2,3-dihydro-3-(1''-imidazolylmethyl)benzo[b]thiophene,
*cis*-5-chloro-3-(2'-chloro-3'-thenyloxy)-2,3-dihydro-2-(1''-imidazolylmethyl)benzo[b]thiophene,
*cis*-7-chloro-3-(2'-chloro-3'-thenyloxy)-2,3-dihydro-2-(1''-imidazolylmethyl)benzo[b]thiophene,
*cis*-5,6-dichloro-3-(2'-chloro-3'-thenyloxy)-2,3-dihydro-2-(1''-imidazolylmethyl)benzo[b]thiophene,
*cis*-5,7-dichloro-3-(2'-chloro-3'-thenyloxy)-2,3-dihydro-2-(1''-imidazolylmethyl)benzo[b]thiophene,
*cis*-6-trifluoromethyl-3-(2'-chloro-3'-thenyloxy)-2,3-dihydro-2-(1''-imidazolylmethyl)-benzo[b]thiophene,
*cis*-3-(4'-chlorobenzyloxy)-2,3-dihydro-2-(1''-imidazolylmethyl)benzo[b]thiophene,

*cis*-5-chloro-3-(4'-chlorobenzyloxy)-2,3-dihydro-2-(1''-imidazolylmethyl)benzo[b]thiophene,
*cis*-5-chloro-3-(2',4'-dichlorobenzyloxy)-2,3-dihydro-2-(1''-imidazolylmethyl)benzo[b]thiophene,
*cis*-4,6-dichloro-3-(4'-chlorobenzyloxy)-2,3-dihydro-2-(1''-imidazolylmethyl)benzo[b]thiophene,
*cis*-4,6-dichloro-3-(2',4'-dichlorobenzyloxy)-2,3-dihydro-2-(1''-imidazolylmethyl)benzo[b]thiophene,
*cis*-5,6-dichloro-3-(2',4'-dichlorobenzyloxy)-2,3-dihydro-2-(1''-imidazolylmethyl)benzo[b]thiophene,
*cis*-5-chloro-3-(2',6'-dichlorobenzyloxy)-2,3-dihydro-2-(1''-imidazolylmethyl)benzo[b]thiophene,
*cis*-6-chloro-3-(2'-chloro-3'-thenyloxy)-2,3-dihydro-2-(1''-imidazolylmethyl)benzo[b]thiophene,
*cis*-5-chloro-3-[2'-(4''-(1'''-acetyl-4'''-piperazinyl)-phenoxy)ethoxy]-2,3-dihydro-2-(1''''-imidazolylmethyl)benzo[b]thiophene, and
*cis*-6-chloro-3-(3'-pyridylmethoxy)-2,3-dihydro-2-(1''-imidazolylmethyl)benzo[b]thiophene.

Other typical compounds of formula I include:

*cis*-6-chloro-3-(2'-chloro-3'-thenyloxy)-2,3-dihydro-2-[1''-(2''-methylimidazolyl)methyl]benzo[b]thiophene,
*cis*-6-chloro-3-(*n*-hexyloxy)-2,3-dihydro-2-(1'-imidazolylmethyl)benzo[b]thiophene,
*cis*-6-chloro-3-(cyclopropylmethoxy)-2,3-dihydro-2-(1'-imidazolylmethyl)benzo[b]thiophene,
*cis*-6-chloro-3-(2',4'-dichlorobenzyloxy)-2,3-dihydro-2-(1''-imidazolylmethyl)benzo[b]thiophene-1-oxide,
*cis*-6-chloro-(2',4'-dichlorobenzyloxy)-2,3-dihydro-2-(1''-imidazolylmethyl)benzo[b]thiophene-1,1-dioxide,
*cis*-7-chloro-3-(methylthiomethoxy)-2,3-dihydro-2-(1''-imidazolylmethyl)benzo[b]thiophene,
*cis*-7-chloro-4-(4'-chlorobenzyloxy)-3-(1''-imidazolylmethyl)thiochroman,
*cis*-7-chloro-4-(2'-chloro-3'-thenyloxy)-3-(1''-imidazolylmethyl)thiochroman,
*cis*-4-(2',6'-dichlorobenzyloxy)-3-(1''-imidazolylmethyl)thiochroman,
*cis*-7-trifluoromethyl-4-(2',5'-dichloro-3'-thenyloxy)-3-(1''-imidazolylmethyl)thiochroman, and
*cis*-7-chloro-4-(4'-chlorobenzyloxy)-3-[1''-(2''-methylimidazolyl)methyl]thiochroman.

Compounds of the formula I in which X is oxygen include:

*cis*-6-chloro-3-(2'-chloro-3'-thenyloxy)-2,3-dihydro-2-(1''-imidazolylmethyl)benzo[b]furan,
*cis*-6-chloro-3-(2'-chloro-3'-thenyloxy)-2,3-dihydro-2-[1''-(2''-methylimidazolyl)methyl]benzo[b]furan,
*cis*-7-chloro-4-(4'-chlorobenzyloxy)-3-(1''-imidazolylmethyl)chroman, and
*cis*-7-chloro-4-(2'-chloro-5'-thenyloxy)-3-(1''-imidazolylmethyl)chroman.

Compounds of formula I are basic and can be converted into acid addition salts by reaction with acids, in particular by at least neutralising the free base of the formula I, preferably by reaction to about pH 5. Particularly suitable acids for this purpose include hydrochloric, sulfuric, phosphoric, hydrobromic and nitric acids.

Compounds of the formula I and their acid acid addition salts can be prepared by methods that are in general known for compounds having similar structures. According to another feature of the invention, we provide a process for the preparation of a compound of the formula I or acid addition salt thereof, which comprises reacting a compound of the formula

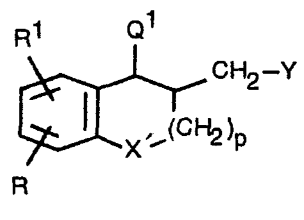

III

with a compound of the formula

$$Q^2—(CH_2)_n—Z \qquad IV$$

wherein R, $R^1$, Y, Z, *n* and *p* are as defined for formula I, X' is an oxygen or sulfur atom, and one of $Q^1$ and $Q^2$ is a hydroxy group and the other is a reactive ester group, in the presence of an inert organic solvent and of an alkali metal base;
whereafter, (i) for the preparation of a compound of the formula I wherein X is the group SO or $SO_2$ when X in the resulting product is S, said product is oxidised;
and (ii) for the preparation of a compound of the formula I wherein the broken line indicates a double bond, a resulting product wherein X is SO is dehydrated;
and the compound of the formula I is isolated as the free base or as an acid addition salt thereof.

The reactive ester group represented by $Q^1$ or more preferably by $Q^2$ can be a hydrocarbonsulfonyloxy group but is preferably a halogen atom, in particular chlorine or bromine.

This process is preferably carried out by allowing a compound of the formula III in which $Q^1$ is a hydroxy group to react with an alkali metal base, e.g. an alkali metal hydride such as sodium hydride, an alkali metal hydroxide such as potassium hydroxide, an alkali metal amide or an alkali metal alcoholate, in

an inert anhydrous organic solvent and then adding the halide of the formula IV wherein $Q^2$ is a halogen atom. The organic solvent may for example be an amide (e.g. dimethylformamide or hexamethylphosphoric acid triamide), an aromatic hydrocarbon (e.g. benzene or toluene), an ether (e.g., diethyl ether, dioxan, tetrahydrofuran or ethylene glycol dimethyl ether), a ketone (e.g. acetone) or, if the base is an alkali metal alcoholate, a lower alkanol (e.g. methanol or ethanol). The reaction with the halide of the formula IV can conveniently be carried out at 0—100°C., preferably at 20—60°C., e.g. at ambient temperature.

In order to increase the yield of product of the formula I, the alkali metal base and halide may be used in excess.

In a particularly preferred method of preparation of compounds of formula I where X is sulfur, sodium hydride is added to a solution of a compound of formula III wherein $Q^1$ is a hydroxy group in dimethylformamide at 0—5°C., and, after reaction at room temperature for about an hour, the halide $Q^2(CH_2)_nZ$ (wherein $Q^2$ is a bromine or chlorine atom and *n* and Z are as defined for formula I) is added and the reaction mixture is left for about a further hour at room temperature. The resulting compound of formula I is isolated and purified by known techniques such as extraction, chromatography, and recrystallization.

Compounds of formula I wherein X is SO (sulfoxides) or $SO_2$ (sulfones) are then prepared by oxidation of compounds of formula I wherein X is sulfur, preferably with an organic peracid in an inert organic solvent, especially with *m*-chloroperbenzoic acid (approximately an equimolar quantity to provide the sulfoxide and double the equimolar quantity to provide the sulfone) in a halogenated hydrocarbon such as chloroform or methylenechloride, at room temperature or preferably below, e.g. at 0—5°C. Compounds wherein X is a sulfur atom and in which the broken line indicates a double bond are prepared by dehydration of a compound of the formula I in which X is SO, *p* is 0 and the broken line indicates a single bond, for example by means of an acid anhydride such as acetic anhydride or especially trifluoroacetic anhydride, e.g. at elevated temperature in an inert organic solvent such as an aromatic hydrocarbon, especially benzene or toluene, and in particular under reflux.

The halide starting materials, $Q^2(CH_2)_nZ$, in which $Q^2$ is a halogen atom and *n* and Z are as defined above, either are already known or can be prepared by standard methods. Examples of typical halides useful in this first process according to the invention include *n*-hexyl chloride, allyl chloride, preopargyl chloride, cyclopropylmethyl chloride, methoxymethyl chloride, methylthioethyl chloride, *p*-chlorophenoxymethyl chloride, p-chloro-phenylthiomethyl chloride, and 1-{[4'-(1''-acetyl-4''-piperazinyl)]-phenoxy}-2-bromoethane.

The starting materials of formula III where X is sulfur may be made by one of the following standard sequences of reactions:

(a) a thiochroman-4-one substituted in the benzene nucleus with R and $R^1$ groups as defined for formula I is first converted into the corresponding α-bromoketone by reaction with bromine in a solvent such as ether, chloroform, or carbon tetrachloride. The resulting α-bromoketone is then reduced to the corresponding bromohydrin, e.g. by reaction with sodium borohydride in a solvent such as a lower alcohol, especially methanol or ethanol. This bromohydrin is then reacted with a compound of the formula YH (wherein Y is as defined above), e.g. with imidazole or a substituted imidazole, in a solvent such as dimethylformamide, hexamethylphosphoric acid triamide, a lower alcohol or acetonitrile to give a compound of formula III wherein q is 1 and *p* is 0, e.g. a 2-imidazolylmethylbenzo[b]thiophene; i.e., the 6-membered heterocyclic ring of the thiochroman has contracted to a 5-membered ring.

(b) An aqueous solution of a compound of the formula YH (wherein Y is as defined above), e.g. imidazole or a substituted imidazole, is reacted with a 3-dimethylamino-methyl-thiochroman-4-one. The resulting 3-Y—$CH_2$-thiochroman-4-one is reduced, e.g. with sodium borohydride in a lower alkanol, to yield a compound of formula III as an isomeric mixture of *cis-* and *trans-*forms; this compound may then be used as a mixture in the above process according to the invention or be separated into the pure *cis-* and *trans-*forms by standard methods, e.g. by chromatography.

The compounds of formula I and their salts exhibit a wide range of antimicrobial activity. Thus, they exhibit antifungal activity against fungal pathogens of humans and animals, such as *Aspergillus, Candida, Epidermophyton, Geotrichum, Microsporum, Monosporium, Pityrosporum, Rhodotorula, Saccharomyces, Trichophyton, Trichosporon,* and *Torulopsis;* antiprotozoal activity against protozoal pathogens such as *Trichomonas;* and antibacterial activity against bacterial pathogens of humans and animals such as *Actinomyces, Bacillus, Bacteroides, Clostridium, Escherichia, Mycobacterium, Nocardia, Propionibacterium, Sarcina, Staphylococcus, Streptococcus,* and *Streptomyces.* They also exhibit activity against fungi of primarily agricultural significance such as *Cladosporium, Colletotrichum, Erysiphe, Fusarium, Helminthosporium, Penicillium, Peronospora, Phytophthora, Pithomyces, Polyspora, Puccina, Rhizoctonia, Sclerotium, Uromyces,* and *Venturia,* and against bacteria of primarily agricultural significance such as *Agrobacterium, Erwinia,* and *Xanthemonas.*

The following Preparations show how the starting materials for the processes according to the invention (especially those of formula III) can be obtained:

Preparation 1

*cis*-2,3-dihydro-3-hydroxy-2-(1'-imidazolylmethyl)-benzo]b]thiophenes

A) *cis*-6-Chloro-2,3-dihydro-3-hydroxy-2-(1'-imidazolylmethyl)benzo]b]thiophene

*1) 3-Bromo-7-chlorothiochroman-4-one*

Dissolve 7-chlorothiochroman-4-one (10 g., 50.3 mmole) in chloroform (100 ml.) and cool the solution to 0—5°C. Add bromine (2.60 ml., 50.3 mmole) dropwise over a 10-minute period. Stir the reaction mixture at room temperature for one hour, then add chloroform (100 ml.) and extract with 10% aqueous sodium sulfite (100 ml.) followed by water (200 ml.). Dry the chloroform solution over anhydrous magnesium sulfate, filter and evaporate *in vacuo* to a residue. Recrystallize the residue from cyclohexane to give 3-bromo-7-chlorothiochroman-4-one, m.p. 109—110°C.

*2) 3-Bromo-7-chlorothiochroman-4-ol*

Suspend 3-bromo-6-chlorothiocroman-4-one (59.6 g., 215 mmole) in methanol (500 ml.), cool to 0—5°C., and with stirring add sodium borohydride (8.18 g., 215 mmole) in three portions. After stirring the reaction mixture at room temperature for three hours, pour it into ice water (4 liters) and extract with chloroform (2 liters). Dry the chloroform solution over anhydrous magnesium sulfate, filter and evaporate *in vacuo* to a residue. Triturate the residue with chloroform/hexane to give 3-bromo-7-chlorothiochroman-4-ol, m.p. 141—142°C.

*3)* cis*-6-chloro-2,3-dihydro-3-hydroxy-2-(1'-imidazolylmethyl)benzo[b]thiophene*

Add 3-bromo-6-chlorothiochroman-4-ol (5.27 g., 18.8 mmole) and imidazole (12.8 g., 188 mmole) to acetonitrile (100 ml.), and reflux for 4 hours. Pour the reaction mixture into water (500 ml.), and extract with chloroform (500 ml.). Wash the organic layer with water (500 ml.) dry it over anhydrous magnesium sulfate, filter and evaporate *in vacuo.* Triturate the residue with anhydrous ether, filter and recrystallize from acetonitrile to give *cis*-6-chloro-2,3-dihydro-3-hydroxy-1-(1'-imidazolylmethyl)-benzo[b]thiophene.

B) In the procedure of above Preparation 1A (1—3), substitute for the 7-chlorothiochroman-4-one an equivalent quantity of each of the following:

a) thiochroman-4-one,
b) 6-chlorothiochroman-4-one,
c) 8-chlorothiochroman-4-one,
d) 5,7-dichlorothiochroman-4-one,
e) 6,7-dichlorothiochroman-4-one,
f) 6,8-dichlorothiochroman-4-one,
g) 7-trifluoromethylthiochroman-4-one, to obtain, respectively,
a) *cis*-2,3-dihydro-3-hydroxy-2-(1'-imidazolylmethyl)benzo[b]thiophene,
b) *cis*-5-chloro-2,3-dihydro-3-hydroxy-2-(1'-imidazolylmethyl)benzo[b]thiophene,
c) *cis*-7-chloro-2,3-dihydro-3-hydroxy-2-(1''-imidazolylmethyl)benzo[b]thiophene,
d) *cis*-4,6-dichloro-2,3-dihydro-3-hydroxy-2-(1''-imidazolylmethyl)benzo[b]thiophene,
e) *cis*-5,6-dichloro-2,3-dihydro-3-hydroxy-2-(1''-imidazolylmethyl)benzo[b]thiophene,
f) *cis*-5,6-dichloro-2,3-dihydro-3-hydroxy-2-(1''-imidazolylmethyl)benzo[b]thiophene,
g) *cis*-6-trifluoromethyl-2,3-dihydro-3-hydroxy-2-(1''-imidazolylmethyl)benzo[b]thiophene.

Preparation 2

3-(1'-imidazolylmethyl)thiochroman-ols

A) 7-Chloro-3-(1'-imidazolylmethyl)thiochroman-4-ol

*1) 7-Chloro-1-3-(1'-imidazolylmethyl;thiochroman-4-one*

Dissolve imidazole (25.8 g., 380 mmole) in water (100 ml.; and add 7-chloro-3-(dimethylaminomethyl)-thiochroman-4-one hydrochloride (11.1 g., 38.0 mmole). Stir the reaction mixture overnight at room temperature, then add water (1 liter) and extract with chloroform (500 ml.). Wash the chloroform solution with 2 liter portions of water, dry it over anhydrous magnesium sulfate, filter and evaporate *in vacuo* to a residue.

Chromatograph the residue on a silica gel column eluting with chloroform. Combine like eluates as determined by thin layer chromatography and evaporate *in vacuo* to a residue comprising 7-chloro-3-(1'-imidazolylmethyl)thiochroman-4-one. Further purify by recrystallization from chloroform/hexane, m.p. 104—105°C.

2) Dissolve 7-chloro-3-(1'imidazolylmethyl)thiochroman-4-one (5.0 g., 17.9 mmole) in methanol (100 ml.), cool the solution to 0—5°C., and add with stirring sodium borohydride (0.51 g., 13.4 mmole). Stir the reaction mixture overnight at room temperature and evaporate *in vacuo.* Add water (100 ml.) to the residue and stir for 30 minutes. Filter and wash the residue with water (2 × 200 ml.) and chromatograph on a silica gel column eluting with chloroform/methanol/ammonium hydroxide (97:3:0.1). Combine like fractions as determined by thin layer chromatography and evaporate the combined like eluates to give two isolated products, namely,

a) *cis*-7-chloro-3-(1'-imidazolylmethyl)thiochroman-4-ol, m.p. 210—212°C. (from ethanol), and
b) *trans*-7-chloro-3-(1'-imidazolylmethyl)-thiochroman-4-ol, m.p. 135°C. (from ethanol).

B) In the procedure of Preparation 2A, by substituting for the 7-chloro-3-(dimethylaminomethyl)-thiochroman-4-one hydrochloride an equivalent quantity of each of the following:

a) 3-(dimethylaminomethyl)thiochroman-4-one hydrochloride,
b) 6-chloro-3-(dimethylaminomethyl)thiochroman-4-one hydrochloride,
c) 8-chloro-3-(dimethylaminomethyl)thiochroman-4-one hydrochloride,
d) 5,7-dichloro-3-(dimethylaminomethyl)thiochroman-4-one hydrochloride,
e) 6,7-dichloro-3-(dimethylaminomethyl)thiochroman-4-one hydrochloride,
f) 6,8-dichloro-3-(dimethylaminomethyl)thiochroman-4-one hydrochloride,
g) 7-trifluoromethyl-3-(dimethylaminomethyl)thiochroman-4-one hydrochloride.

there is obtained, upon reaction with imidazole in the manner of Preparation 2A(1), the corresponding 3-(1'-imidazolyl-methyl)thiochroman-4-one derivative, which, upon reaction with sodium borohydride according to Preparation 2A(2), yields the corresponding 4-hydroxyl derivative which, upon purification by chromatography in the described manner, is separated into its *cis* and *trans* isomers, i.e.,

a) *cis-* and *trans*-3-(1'-imidazolylmethyl)thiochroman-4-ol,
b) *cis-* and *trans*-6-chloro-3-(1'-imidazolylmethyl)thiochroman-4-ol,
c) *cis-* and *trans*-8-chloro-3-(1'-imidazolylmethyl)thiochroman-4-ol,
d) *cis-* and *trans*-5,7-dichloro-3-(1'-imidazolylmethyl)thiochroman-4-ol,
e) *cis-* and *trans*-6,7-dichloro-3-(1'-imidazolylmethyl)thiochroman-4-ol,
f) *cis-* and *trans*-6,8-dichloro-3-(1'-imidazolylmethyl)thiochroman-4-ol, and
g) *cis-* and *trans*-7-trifluoromethyl-3-(1'-imidazolylmethyl)thiochroman-4-ol.

### Preparation 3

1-{[4'-(1''-acetyl-4''-piperazinyl)]phenoxy}-2-bromoethane

Dissolve 1-acetyl-4-(4'-hydroxyphenyl)piperazine (15.0 g., 58.5 mmole) and potassium hydroxide (3.28 g., 58.5 mmole) in absolute ethanol (500 ml.). Add ethylenedibromide (50.4 ml., 585 mmole) and reflux the mixture for two hours. Add another portion of potassium hydroxide (3.28 g., 58.5 mmole) and reflux again for two hours; repeat this last step two more times. Evaporate the ethanol *in vacuo.* Dissolve the resulting residue in chloroform (1 liter) and extract with water (1 liter). Dry the chloroform solution over anhydrous magnesium sulfate, evaporate, and chromatograph the residue on silica gel, eluting with chloroform. Combine like fractions as determined by thin layer chromatography and evaporate them to give 1-{[4'-(1''-acetyl-4-''-piperazinyl)]phenoxy}-2-bromoethane.

### Preparation 4

5-chloro-2,3-dihydro-3-hydroxy-2-(1'-imidazolylmethyl)benzo[b]furan

A) 5-chloro-2-(1'-imadzolylmethyl)coumaran-3-one

Dissolve imidazole (37.44 g., 550 mmole) in water (200 ml.) and add 5-chloro-2-(dimethylaminomethyl)coumaran-3-one hydrochloride (25 g. 55 mmole). Stir the reaction mixture overnight at room temperature, then add water (2 liters) and extract with chloroform (1 liter). Wash the chloroform solution with another 2 liter portion of water, dry over anhydrous magnesium sulfate, filter and evaporate *in vacuo* to give 5-chloro-2-(1'-imidazolylmethyl)coumaran-3-one.

B) 5-chloro-2,3-dihydro-3-hydroxy-2-(1'-imidazolylmethyl)benzo[b]furan

Dissolve 5-chloro-2-(1'-imidazolymethyl)coumaran-3-one (10 g., 21 mmole) in methanol (100 ml.), cool the solution to 0—5°C. and add sodium borohydride (1.0 g., 26.4 mmole). Stir the reaction mixture overnight at room temperature and evaporate *in vacuo*. Add water (200 ml.) to the residue and stir for 30 minutes. Filter and wash the residue with water to give 5-chloro-2,3-dihydro-3-hydroxy-2-(1'-imidazolylmethyl)benzo[b]furan.

### Preparation 5

Subject equivalent quantities of each of 7-fluoro-thiochroman-4-one and 8-fluoro-thiochroman-4-one to the series of reactions described in Preparation 1A(1—3) to obtain *cis*-6-fluoro-2,3-dihydro-3-hydroxy-2-(1'-imidazolymethyl)benzo[b]thiophene and *cis*-7-fluoro-2,3-dihydro-3-hydroxy-2-(1'-imidazolylmethyl)-benzo[b]thiophene.

The following Examples illustrate the preparation of compounds of formula I and of their acid addition salts; a number in square brackets, e.g. [390], indicates a characteristic mass spectrum [$M^+$] peak.

### Example 1

*Cis*-3-chlorothenyloxy (or chlorobenzyloxy) -2,3-dihydro-2-(1''-imidazolymethyl)benzo[b]thiophenes

A. *cis*-6-Chloro-3-(2'-chloro-3'-thenyloxy)-2,3-dihydro-2-(1''-imidazolylmethyl)benzo[b]thiophene

To a solution of *cis*-6-chloro-2,3-dihydro-3-hydroxy-2-(1''-imidazolylmethyl)benzo[b] thiophene (200 g., 7.50 mmole) in dry dimethylformamide (20 ml.) cooled to 0—5°C., add sodium hydride (50% dispersion, 0.40 g., 8.25 mmole) and stir at room temperature for one hour. Add 2-chloro-3-thenyl bromide (1.75 g., 8.25 mmole) and stir at room temperature for another hour. Pour the reaction mixture into ether (500 ml.) and extract with three 500 ml. portions of water. Dry the ether solution over anhydrous magnesium sulfate, filter and evaporator *in vacuo*. Chromatograph the residue on silica gel eluting with chloroform. Combine like eluates as determined by thin layer chromatography, evaporate them *in vacuo* and recrystallize the residue from cyclohexane to obtain *cis*-6-chloro-3-(2'-chloro-3'-thenyloxy)-2,3-dihydro-2-(1''-imidazolylmethyl)-benzo[b]thiophene, m.p. 99—101°C.

B. In the procedure of Example 1A, instead of 2-chloro-3-thenyl bromide, use an equivalent quantity of each of the following reagents:

a) 2,5-dichloro-3-thenyl bromide,

b) 4-chlorobenzyl chloride,

c) 2,4-dichlorobenzyl chloride, to obtain, respectively,

*cis*-6-chloro-3-(2',5'-dichloro-3'-thenyloxy)-2,3-dihydro-2-(1''imidazolylmethyl)benzo[b]thiophene,

*cis*-6-chloro-3-(4'-chlorobenzyloxy)-2,3-dihydro-2-(1''-imidazolylmethyl)benzo[b]thiophene,[390], and

*cis*-6-chloro-3-(2',4'-dichlorobenzyloxy)-2,3-dihydro-2-(1''-imidazolymethyl)benzo[b]thiophene, m.p. 122—124°C.

C. Treat each of the *cis*-2,3-dihydro-3-hydroxy-2-(1''-imidazolylmethylbenzo[b]thiphenes prepared in Preparation 1B with 2-chloro-3-thenyl bromide in a manner similar to that described in Example 1A to obtain, respectively,

a) *cis*-3-(2'-chloro-3'-thenyloxy)-2,3-dihydro-2-(1''-imidazolylmethyl)benzo[b]thiophene, m.p. 101—102°C.,

b) *cis*-5-chloro-3-(2'-chloro-3'-thenyloxy)-2,3-dihydro-2-(1''-imidazolylmethyl)benzo[b]thiophene, m.p. 95—97°C.,

c) *cis*-7-chloro-(2'-chloro-3'-thenyloxy)-2,3-dihydro-2-(1''-imidazolylmethyl)benzo[b]thiophene, m.p. 86—87°C.,

d) *cis*-4,6-dichloro-3-(2'-chloro-3'-thenyloxy)-2,3-dihydro-2-(1''-imidazolylmethyl)benzo[b]thiophene,

e) *cis*-5,6-dichloro-3-(2'-chloro-3'-thenyloxy)-2,3-dihydro-2-(1''-imidazolylmethyl)benzo[b]thiophene, m.p. 128—129°C.,

f) *cis*-5,7-dichloro-3-(2'-chloro-3'-thenyloxy)-2,3-dihydro-2-(1''-imidazolylmethyl)benzo[b]thiophene, and

g) *cis*-6-trifluoromethyl-3-(2'-chloro-3'-thenyloxy)-2,3-dihydro-2-(1''-imidazolylmethyl)benzo[b]-thiophene, m.p. 112—114°C.

D. Similarly, treat each of the *cis*-2,3-dihydro-3-hydroxy-(1''-imidazolylmethyl)benzo[b]thiophenes prepared in Preparation 1B with each of the three reagents listed in Example 1B to obtain respectively,

a)1) *cis*-3-(2',5'-dichloro-3-thenyloxy)-2,3-dihydro-2-(1''-imidazolylmethyl)benzo[b]thiophene, [356],

2) *cis*-3-(4'-chlorobenzyloxy)-2,3-dihydro-2-(1''-imidazolylmethyl)benzo[b]thiophene,

3) *cis*-(2',4'-dichlorobenzyloxy)-2,3-dihydro-2-(1''-imidazolylmethyl)benzo[b]thiophene,

b)1) *cis*-5-chloro-3-(2',5'-dichloro-3'-thenyloxy)-2,3-dihydro-2-(1''-imidazolylmethyl)benzo[b]-thiophene,

2) *cis*-5-chloro-3-(4'-chlorobenzyloxy)-2,3-dihydro-2-(1''-imidazolylmethyl)benzo[b]thiophene, m.p. (nitrate) 157—158°C.,

3) *cis*-5-chloro-3-(2',4'-dichlorobenzyloxy)-2,3-dihydro-2-(1''-imidazolylmethyl)benzo[b]thiophene, m.p. 95—97°C.;

c)1) *cis*-7-chloro-3-(2',5'-dichloro-3'-thenyloxy)-2,3-dihydro-2-(1''-imidazolylmethyl)benzo[b]thiophene,

2) *cis*-7-chloro-3-(4'-chlorobenzyloxy)-2,3-dihydro-2-(1''-imidazolylmethyl)benzo[b]thiophene,

3) *cis*-7-chloro-3-(2',4'-dichlorobenzyloxy)-2,3-dihydro-2-(1''-imidazolylmethyl)benzo[b]thiophene;

d)1) *cis*-4,6-dichloro-3-(2',5'-dichloro-3'-thenyloxy)-2,3-dihydro-2-(1''-imidazolylmethyl)benzo[b]-thiophene,

2) *cis*-4,6-dichloro-3-(4'-chlorobenzyloxy)-2,3-dihydro-2-(1''-imidazolylmethyl)benzo[b]thiophene, m.p. 147—149°C.,

3) *cis*-4,6-dichloro-3-(2',4'-dichlorobenzyloxy)-2,3-dihydro-2-(1''-imidazolylmethyl)benzo[b]thiophene, m.p. 122—124°C.;

e)1) *cis*-5,6-dichloro-3-(2',5'-dichloro-3'-thenyloxy)-2,3-dihydro-2-(1''-imidazolylmethyl)benzo[b]-thiophene,

2) *cis*-5,6-dichloro-3-(4'-chlorobenzyloxy)-2,3-dihydro-2-(1''-imidazolylmethyl)benzo[b]thiophene,

3) *cis*-5,6-dichloro-3-(2',4'-dichlorobenzyloxy)-2,3-dihydro-2-(1''-imidazolylmethyl)benzo[b]-thiophene,;

f)1) *cis*-5,7-dichloro-3-(2',5'-dichloro-3'-thenyloxy)-2,3-dihydro-2-(1''-imidazolylmethyl)benzo[b]-thiophene,

2) *cis*-5,7-dichloro-3-(4'-chlorobenzyloxy)-2,3-dihydro-2-(1''-imidazolylmethyl)benzo[b]thiophene,

3) *cis*-5,7-dichloro-3-(2',4'-dichlorobenzyloxy)-2,3-dihydro-2-(1''-imidazolylmethyl)benzo[b]thiophene;

g)1) *cis*-6-trifluoromethyl-3-(2',5'-dichloro-3'-thenyloxy)-2,3-dihydro-2-(1''-imidazolylmethyl)benzo[b]-thiophene,

2) *cis*-6-trifluoromethyl-3-(4'-chlorobenzyloxy)-2,3-dihydro-2-(1''-imidazolylmethyl)benzo[b]-thiophene, and

3) *cis*-6-trifluoromethyl-3-(2',4'-dichlorobenzyloxy)-2,3-dihydro-2-(1''-imidazolylmethyl)benzo[b]-thiophene.

E. Treat each of *cis*-6-fluoro-2,3-dihydro-3-hydroxy-2-(1'-imidazolylmethyl)benzo[b]thiophene and *cis*-5-fluoro-2,3-dihydro-3-hydroxy-2-(1'-imidazolylmethyl)benzo[b]thiophene (from Preparation 5) in a manner described in each of Examples 1A and 1B to obtain, respectively:

a)1) *cis*-6-fluoro-3-(2'-chloro-3'-thenyloxy)-2,3-dihydro-2-(1''-imidazolylmethyl)benzo[b]thiophene, m.p. 76—78°C.,

2) *cis*-6-fluoro-3-(2',5'-dichloro-3'-thenyloxy)-2,3-dihydro-2-(1''-imidazolylmethyl)benzo[b]thiophene,

3) *cis*-6-fluoro-3-(4'-chlorobenzyloxy)-2,3-dihydro-2-(1''-imidazolylmethyl)benzo[b]thiophene,

4) *cis*-6-fluoro-3-(2',4'-dichlorobenzyloxy-2,3-dihydro-2-(1''-imidazolylmethyl)benzo[b]thiophene;

b)1) *cis*-5-fluoro-4-(2'-chloro-3'-thenyloxy)-2,3-dihydro-2-(1''-imidazolylmethyl)benzo[b]thiophene, m.p. 109—111°C.,

2) *cis*-5-fluoro-3-(2',5'-dichloro-3'-thenyloxy)-2,3-dihydro-2-(1''-imidazolylmethyl)benzo[b]thiophene,

3) *cis*-5-fluoro-3-(4'-dichlorobenzyloxy)-2,3-dihydro-2-(1''-imidazolylmethyl)benzo[b]thiophene, and

4) *cis*-5-fluoro-3-(2',4'dichlorobenzyloxy)-2,3-dihydro-2-(1''-imidazolylmethyl)benzo[b]thiophene.

## Example 2

4-chlorobenzyloxy (or 2-chloro-3-thenyloxy) -3-(1'-imidazolylmethyl)thiochromans

A. *cis*-7-Chloro-4-(4'-chlorobenzyloxy)-3-(1''-imidazolylmethyl)-thiochroman is prepared by the method of Example 1A from *cis*-7-chloro-3-(1'-imidazolylmethyl)-thiochroman-4-ol (1.59 g., 5.66 mmole), sodium hydride (50% dispersion) (0.34 g., 7.0 mmole), dry dimethylformamide (10 ml.) and *p*-chlorobenzyl chloride (1.13 g., 7.0 mmole), except that the reaction mixture is poured into chloroform (100 ml.).

To prepare the nitrate salt, dissolve the free base (a gummy residue) in anhydrous ether (250 ml.) and add dropwise a solution of nitric acid in 2-propanol until precipitation is complete. Filter off the resulting precipitate of *cis*-7-chloro-4-(4'-chlorobenzyloxy)-3-(1''-imidazolylmethyl)thiochroman nitrate and recrystallize it from ethyl acetate/hexane, m.p. 135—137°C.

B. *trans*-7-Chloro-4-(4'-chlorobenzyloxy)-3-(1''-imidazolylmethyl)-thiochroman can be prepared similarly from *trans*-7-chloro-3-(1'-imidazolylmethyl)-thiochroman-4-ol (0.5 g., 1.78 mmole), sodium hydride (50% dispersion in oil) (0.11 g., 2.29 mmole) in dry dimethylformamide (10 ml.) and *p*-chlorobenzyl chloride (0.36 g., 2.23 mmole), [404].

C. In the procedure of each of Examples 2A and 2B replace *p*-chlorobenzyl chloride with an equivalent quantity of 2-chloro-3-thenyl bromide to obtain, respectively, *cis*-7-chloro-4-(2'-chloro-3'-thenyloxy)-3-(1''-imidazolylmethyl)thiochroman, [378], and *trans*-7-chloro-4-(2'-chloro-3'thenyloxy)-3-(1''-imidazolylmethyl)-thiochroman.

D. Treat each of the 3-(1'-imidazolylmethyl)thiochroman-4-ols prepared in Preparation 2B with *p*-chlorobenzyl chloride in the manner of Examples 2A and 2B or with 2,5-dichloro-3-thenyl bromide in the manner of Example 2C to obtain, respectively,

*cis*-4-(4'-chlorobenzyloxy)-3-(1''-imidazolylmethyl)thiochroman, m.p. (nitrate) 136—138°C.;
*cis*-4-(2'-chloro-3'-thenyloxy)-3-(1''-imidazolylmethyl)thiochroman;
*cis*-6-chloro-4-(4'-chlorobenzyloxy)-3-(1''-imidazolylmethyl)thiochroman, [390];
*cis*-6-chloro-4-(2'-chloro-3'-thenyloxy)-3-(1''-imidazolylmethyl)thiochroman;
*cis*-8-chloro-4-(4'-chlorobenzyloxy)-3-(1''-imidazolylmethyl)thiochroman;
*cis*-8-chloro-4-(2'-chloro-3'-thenyloxy)-3-(1''-imidazolylmethyl)thiochroman;
*cis*-5,7-dichloro-4-(4'-chlorobenzyloxy)-3-(1''-imidazolylmethyl)thiochroman;
*cis*-5,7-dichloro-4-(2'-chloro-3'-thenyloxy)-3-(1''-imidazolylmethyl)thiochroman;
*cis*-6,7-dichloro-4-(4'-chlorobenzyloxy)-3-(1''-imidazolylmethyl)thiochroman;
*cis*-6,7-dichloro-4-(2'-chloro-3'-thenyloxy)-3-(1''-imidazolylmethyl)thiochroman;
*cis*-6,8-dichloro-4-(4'-chlorobenzyloxy)-3-(1''-imidazolylmethyl)thiochroman;
*cis*-6,8-dichloro-4-(2'-chloro-3'-thenyloxy)-3-(1''-imidazolylmethyl)thiochroman;
*cis*-7-trifluoromethyl-4-(4'-chlorobenzyloxy)-3-(1''-imidazolylmethyl)thiochroman;
*cis*-7-trifluoromethyl-4-(2'-chloro-3'-thenyloxy)-3-(1''-imidazolylmethyl)thiochroman;
and the corresponding *trans*-isomer of each of the foregoing.

F. Treat each of the 3-(1'-imidazolylmethyl)thiochroman-4-ols prepared in Preparation 2B in the manner of Examples 2A and 2B except that in place of *p*-chlorobenzyl chloride use an equivalent quantity of each of 2,5-dichloro-3-thenyl bromide, 2,4-dichlorobenzyl chloride, and 2,6-dichlorobenzyl chloride, to obtain, respectively,

a)1) *cis*-4-(2',5'-dichloro-3'-thenyloxy)-3-(1''-imidazolylmethyl)thiochroman,
2) *cis*-4-(2',4'-dichlorobenzyloxy)-3-(1''-imidazolylmethyl)thiochroman,
3) *cis*-4-(2',6'-dichlorobenzyloxy)-3-(1''-imidazolylmethyl)thiochroman,
b)1) *cis*-6-chloro-4-(2',5'-dichloro-3'-thenyloxy)-3-(1''-imidazolylmethyl)thiochroman,
2) *cis*-6-chloro-4-(2',4'-dichlorobenzyloxy)-3-(1''-imidazolylmethyl)thiochroman,
3) *cis*-6-chloro-4-(2',6'-dichlorobenzyloxy)-3-(1''-imidazolylmethyl)thiochroman,
c)1) *cis*-8-chloro-4-(2',5'-dichloro-3'-thenyloxy)-3-(1''-imidazolylmethyl)thiochroman,
2) *cis*-8-chloro-4-(2',4'-dichlorobenzyloxy)-3-(1''-imidazolylmethyl)thiochroman,
3) *cis*-8-chloro-4-(2',6'-dichlorobenzyloxy)-3-(1''-imidazolylmethyl)thiochroman,
d)1) *cis*-5,7-dichloro-4-(2',5'-dichloro-3'-thenyloxy)-3-(1''-imidazolylmethyl)thiochroman,
2) *cis*-5,7-dichloro-4-(2',4'-dichlorobenzyloxy)-3-(1''-imidazolylmethyl)thiochroman,
3) *cis*-5,7-dichloro-4-(2',6'-dichlorobenzyloxy)-3-(1''-imidazolylmethyl)thiochroman,
e)1) *cis*-6,7-dichloro-4-(2',5'-dichloro-3'-thenyloxy)-3-(1''-imidazolylmethyl)thiochroman,
2) *cis*-6,7-dichloro-4-(2',4'-dichlorobenzyloxy)-3-(1''-imidazolylmethyl)thiochroman,

3) *cis*-6,7-dichloro-4-(2′,6′-dichlorobenzyloxy)-3-(1″-imidazolylmethyl)thiochroman,
f)1) *cis*-6,8-dichloro-4-(2′,5′-dichloro-3′-thenyloxy)-3-(1″-imidazolylmethyl)thiochroman,
2) *cis*-6,8-dichloro-4-(2′,4′-dichlorobenzyloxy)-3-(1″-imidazolylmethyl)thiochroman,
3) *cis*-6,8-dichloro-4-(2′,6′-dichlorobenzyloxy)-3-(1″-imidazolylmethyl)thiochroman,
g)1) *cis*-7-trifluoromethyl-4-(2′,5′-dichloro-3′-thenyloxy)-3-(1″-imidazolylmethyl)thiochroman,
2) *cis*-7-trifluoromethyl-4-(2′,4′-dichlorobenzyloxy)-3-(1″-imidazolylmethyl)thiochroman, and
3) *cis*-7-trifluoromethyl-4-(2′,6′-dichlorobenzyloxy)-3-(1″-imidazolylmethyl)thiochroman,
and the corresponding *trans*-isomers of the foregoing.

## Example 3

*Cis*-5-chloro-3-{2′-[4″-(1‴-acetyl-4‴-piperazinyl)phenoxy]ethoxy}-2,3-dihydro-2-(1⁗-imidazolylmethyl)-benzo[b]thiophene

A) Stir a mixture of *cis*-5-chloro-2,3-dihydro-3-hydroxy-2-(1′-imidazolylmethyl)benzo[b]thiophene (0.50 g., 1.87 mmole) and potassium hydroxide (0.15 g., 2.2 mmole) in acetone (10 ml.) at room temperature for one hour. Add 1-{[4′-(1″-acetyl-4″-piperazinyl)]phenoxy}-2-bromoethane and stir overnight at room temperature. Add a second portion of potassium hydroxide (0.15 g., 2.2 mmole) and stir overnight; repeat a third time. Pour the mixture into chloroform (500 ml.) and extract with water (500 ml.). Dry the chloroform solution over anhydrous magnesium sulfate and evaporate *in vacuo.* Chromatograph the resulting residue on silica gel eluting with 2% methanol in chloroform. Combine like fractions as determined by thin layer chromatography and evaporate them to give *cis*-5-chloro-3-{2′-[4″-(1‴-acetyl-4‴-piperazinyl)phenoxy]-ethoxy}-2,3-dihydro-2-(1⁗-imidazolylmethyl)benzo[b]thiophene, [478].

B) Dissolve the product of Step A in a mixture of chloroform (10 ml.) and methanol (10 ml.), acidify to pH 2—3 with hydrochloric acid in 2-propanol, and evaporate to a residue comprising *cis*-5-chloro-3-{2′-[4″-(1‴ - acetyl - 4‴ - piperazinyl)phenoxy]ethoxy} - 2,3 - dihydro - 2 - (1⁗ - imidazolylmethyl)benzo[b]thiophene dihydrochloride.

## Example 4

3-Chlorothenyloxy (or chlorobenzyloxy) -2,3-dihydro-2-(1″-imidazolylmethyl)benzo[b]thiophene-1,-oxides

A) 6-Chloro-3-(2′,4′-dichlorobenzyloxy)-2,3-dihydro-2-(1″-imidazolylmethyl)benzo[b]thiophene-1-oxide

To a solution of 6-chloro-3-(2′,4′-dichlorobenzyloxy)-2,3-dihydro-2-(1″-imidazolylmethyl)benzo[b]-thiophene (0.20 g., 0.5 mmole) in dichloromethane (10 ml.) cooled to 0—5°C., add *m*-chloroperbenzoic acid (0.0812 g., 0.47 mmole) and stir for one hour. Add sodium sulfite (0.05 g., 0.40 mmole) and stir for another 30 minutes. Add dichloromethane (25 ml.) to the solution and extract with 10% aqueous sodium carbonate (25 ml.). Dry the organic phase over anhydrous magnesium sulfate and evaporate. Chromatograph the resulting residue on silica gel eluting with 5% methanol in chloroform. Combine like fractions as determined by thin layer chromatography and evaporate them to give 6-chloro-3-(2′,4′-dichlorobenzyloxy)-2,3-dihydro-2-(1″-imidazolylmethyl)benzo[b]thiophene-1-oxide, m.p. 60°C.

B) Treat each of the *cis*-3-chlorothenyloxy (or chlorobenzyloxy) -2,3-dihydro-2-(1″-imidazolylmethyl)benzo-[b]thiophenes of Example 1, the 4-chlorobenzyloxy- (or 2-chloro-3-thenyloxy)-3-(1″-imidazolylmethyl)-thiochromans of Example 2, and the *cis*-3-{2′-[4‴-(1‴-acetyl-4‴-piperazinyl)phenoxy]ethoxy}-2,3-dihydro-2-(1⁗-imidazolylmethyl)benzo[b]thiophenes of Example 3 in a manner similar to that of Example 4A to obtain the corresponding 1-oxides.

## Example 5

3-Chlorothenoxy (or chlorobenzyloxy -2,3-dihydro-2-1″-imidazolylmethyl)benzo[b]thiophene-1,1-dioxides

A) 6-Chloro-3-(2′,4′-dichlorobenzyloxy)-2,3-dihydro-2-(1″-imidazolylmethyl)benzo[b]thiophene-1,1-dioxide.

To a solution of 6-chloro-3-(2′,4′-dichlorobenzyloxy)-2,3-dihydro-2-(1″-imidazolylmethyl)benzo[b]-thiophene (0.20 g., 0.5 mmole) in dichloromethane (10 ml.) cooled to 0—5°C., add *m*-chloroperbenzoic acid (0.1624 gm., 0.94 mmole) and stir for one hour. Warm the solution to room temperature and stir overnight. Add dichloromethane (25 ml.) and extract with 10% aqueous sodium carbonate (25 ml.). Dry the organic phase over anhydrous magnesium sulfate and evaporate to give a residue comprising 6-chloro-3-(2′,4′-dichlorobenzyloxy)-2,3-dihydro-2-(1″-imidazolylmethyl)benzo[b]thiophene-1,1-dioxide, m.p. 182°C.

B) Treat each of the *cis*-3-chlorothenyl (or chlorobenzyloxy)-2,3-dihydro-2-(1″-imidazolylmethyl)benzo[b]-thiophenes of Example 1, the 4-chlorobenzyloxy (or chlorophenoxy) -3-(1″-imidazolylmethyl)thiochromans of Example 2, and the *cis*-3-{2′-[4″-(1‴-acetyl-4‴-piperazinyl)phenoxy]ethoxy}-2,3-dihydro-2-(1⁗-imidazolylmethyl)benzo[b]thiophenes of Example 3 in a manner similar to that of Example 5A to obtain the corresponding 1,1-dioxides.

## Example 6

*cis*-7-chloro-3-(methylthiomethoxy)-2,3-dihydro-2-(1′-imidazolylmethyl)benzo[b]thiophene, [326] is prepared by the method of Example 1A from *cis*-7-chloro-3-hydroxy-2,3-dihydro-2-(1′-imidazolylmethyl)-benzo[b]thiophene (2.67 g., 10 mmole), dry dimethylformamide (30 ml.), sodium hydride (50% oil dispersion) (0.53 g., 11 mmole) and chloromethyl methyl sulfide (1.06 g., 11 mmole), except that the chromatography is effected by high pressure liquid chromatography using two silica gel cartridges and eluting with ethyl acetate.

Example 7

*cis*-6-chloro-3-(2′-chloro-5′-thenyloxy)-2,3-dihydro-2-(1″-imidazolylmethyl)benzo[b]thiophene, m.p. 94—95°C., is prepared by the method of Example 1A from *cis*-6-chloro-2,3-dihydro-3-hydroxy-2-(1′-imidazolylmethyl)benzo[b]thiophene (2.67 g., 10 mmole), dry dimethylformamide (27 ml.), sodium hydride (50% oil dispersion) (0.53 g., 11 mmole) and 2-chloro-5-thenyl bromide (2.33 g., 11 mmole).

Example 8

*cis*-6-chloro-3-(2′-chloro-3′-thenyloxy)-2,3-dihydro-2-(1″-imidazolylmethyl)benzo[b]thiophene-1-oxide

*cis*-6-Chloro-3-(2′-chloro-3′-thenyloxy)-2,3-dihydro-2-(1″-imidazolylmethyl)benzo[b]thiophene (5 g., 12.6 mmole) was dissolved in dichloromethane (100 ml.) and cooled to about 0—5°C. *m*-Chloroperbenzoic acid (2.65 g., 15.4 mmole) was added and the mixture was stirred for one hour. The solution was diluted with dichloromethane (100 ml.) and washed with 10 percent aqueous sodium bicarbonate (2 × 150 ml.) and water (2 × 150 ml.). The organic extract was separated, dried over magnesium sulfate and filtered. The solvent was evaporated and the product, *cis*-6-chloro-3-(2′-chloro-3′-thenyloxy)-2,3-dihydro-2-(1″-imidazolylmethyl)benzo[b]thiophene-1-oxide (4.8 g.), was obtained as a white crystalline solid. It was recrystallized from ethyl acetate; m.p. 125°C. Analysis: found C 48.74%; H 3.4%; N 6.9%, S 15.25%; Cl 17.29%; $C_{17}H_{14}Cl_2N_2O_2S_2$ requires C 49.35%; H 3.38%; N 6.8%; S 15.48%; Cl 17.17%.

The corresponding 5-chloro isomer (a gum) can be prepared similarly.

Example 9

6-chloro-3-(2′-chloro-3′-thenyloxy)-2-(1″-imidazolylmethyl)benzo[b]thiophene

*cis*-6-Chloro-3-(2′-chloro-3′-thenyloxy)-2,3-dihydro-2-(1″-imidazolylmethyl)benzo[b]thiophene-1-oxide (4.5 g., 10.9 mmole) and trifluoroacetic anhydride (6.86 g., 32.7 mmole) were refluxed in toluene (100 ml) for a half hour. The solution was diluted with dichloromethane (200 ml.) and washed with aqueous sodium bicarbonate (150 ml.) and water (150 ml.). The organic extract was dried over anhydrous magnesium sulfate and filtered, and the solvent was evaporated to leave 6-chloro-3-(2′-chloro-3′-thenyloxy)-2-(1″-imidazolylmethyl)benzo[b]thiophene (3.2 g.); m.p. 84°C. (white needles from dioxan). Analysis: found C 51.52%; H 2.97%; N 7.26%; S 15.95%; Cl 17.98%. $C_{17}H_{12}Cl_2N_2OS_2$ requires C 51.58%; H 3.03%; N 7.08%; S 16.18%; Cl 17.7%.

The corresponding 5-chloro isomer (m.p. 124°C.) can be prepared similarly.

Example 10

A. *cis*-3-(2′-chloro-3′-thenyloxy)-2,3,5,6,7,8-hexahydro-2-(1″-imidazolylmethyl)naphtho[2,3-b]thiophene, m.p. 87—88°C., can be prepared by the method of Example 1 from *cis*-3-hydroxy-2,3,5,6,7,8-hexahydro-2-(1′-imidazolylmethyl)naphtho[2,3-b]thiophene (m.p. 193—195°C.), which itself can be prepared from 2,3,6,7,8,9-hexahydro-4H-naphtho[2,3-b]thiopyran-4-one (Krollpfeifer and Schultze, *Chem. Ber.,* vol 56 (1923), pages 1819—1924, compound V) by the method of Preparation 1.

B. *cis*-3-(2′-chloro-3′-thenyloxy)-2,3-dihydro-2-(1″-imidazolylmethyl)naphtho[1,2-b]thiophene, m.p. of hydrochloride 165—166°C., can be prepared by the method of Example 1 from *cis*-3-hydroxy-2,3-dihydro-2-(1′-imidazolylmethyl)naphtho[1,2-b]thiophene (m.p. 162—165°C.), which itself can be prepared from 2,3-dihydro-4H-naphtho[1,2-b]thiopyran-4-one (Krollpfeifer and Schultze, *Chem. Ber.,* vol. 56 (1923), pages 1819—1924, compound II) by the method of Preparation 1.

C. *cis*-3-(2′-chloro-3′-thenyloxy)-2,3,4,5,6,7-hexahydro-2-(1″-imidazolylmethyl)naphtho[3,4-b]thiophene, m.p. 106—108°C., can be prepared by the method of Example 1 from *cis*-3-hydroxy-2,3,4,5,6,7-hexahydro-2-(1′-imidazolylmethyl)naphtho[3,4-b]thiophene (m.p. 180—181°C.), which itself can be prepared from 2,3,5,6,7,8-hexahydro-4H-naphtho[3,4-b]thiopyran-4-one (Krollpfeifer and Schultze, *Chem. Ber.,* vol. 56 (1923), pages 1819—1924, compound VI) by the method of Preparation 1.

Example 11

A. *cis*-6-chloro-3-allyloxy-2,3-dihydro-2-(1′-imidazolylmethyl)benzo[b]thiophene

*cis*-6-Chloro-3-hydroxy-2-(1′imidazolylmethyl)-2,3-dihydrobenzo[b]thiophene (3 g., 0.011 mmole), allyl chloride (3.44 g., 0.045 mmole) and methyl-tricaprylammonium chloride (1 ml.) were stirred with 10 ml. aqueous 50% sodium hydroxide and 25 ml. tetrahydrofuran for two hours. The reaction mixture was diluted with methylene chloride (200 ml.) and washed with water (4 × 200 ml.). The extract was dried over magnesium sulfate and the solvent was evaporated to give *cis*-6-chloro-3-allyloxy-2,3-dihydro-2-(1′-imidazolylmethyl)benzo[b]thiophene as an oil (3.2 g.).

B. *cis*-6-chloro-3-(quinolyl-2′-methoxy)-2,3-dihydro-2-(1″-imidazolylmethyl)benzo[b]thiophene (a gum) may be prepared similarly by substitution of 2-chloromethylquinoline for allyl chloride in Part A; [407].

C. *cis*-6-chloro-3-(2′-chlorobenzo[b]thienyl-3′-methoxy)-2,3-dihydro-2-(1″-imidazolylmethyl)benzo[b]-thiophene, m.p. 129—130°C., can be prepared similarly by substitution of 3-bromomethyl-2-chlorobenzo-[b]thiophene for allyl chloride in Part A.

3-Bromomethyl-2-chlorobenzo[b]thiophene can be prepared as follows:

A mixture of 2-chloro-3-methylbenzo[b]thiophene (21.9 g., 0.12 mmole), N-bromosuccinimide (21.4 g.,

0.12 mmole) and benzoylperoxide (1.0 g.) in carbon tetrachloride (200 ml.) was refluxed for eight hours. The succinimide was filtered off and the carbon tetrachloride was evaporated. The residue was recrystallized from methanol (with charcoal) to give 3-bromomethyl-2-chlorobenzo[b]thiophene, m.p. 69—70°C.

D. *cis*-6-chloro-3-(4'-thiazolylmethoxy)-2,3-dihydro-2-(1''-imidazolylmethyl)benzo[b]thiophene, m.p. 92—93°C., can be prepared similarly by substitution of 4-chloromethylthiazole (Caldwell and Fox, *J.A.C.S., 73,* pages 2395—6 (1951)) for allyl chloride in Part A.

E. The following compounds can be prepared similarly:

*cis*-6-chloro-3-(6'-chloro-3',4'-methylenedioxybenzyloxy)-2,3-dihydro-2-(1''-imidazolylmethyl)benzo[b]thiophene, m.p. 130—132°C.;

*cis*-6-chloro-3-(2'-chloro-6'-fluorobenzyloxy)-2,3-dihydro-2-(1''-imidazolylmethyl)benzo[b]thiophene, m.p. 140°C.;

*cis*-6-chloro-3-[(5'-chloro-3'-methyl-1'-phenyl-pyrazol-4'-yl)methoxy]-2,3-dihydro-2-(1''-imidazolylmethyl)benzo[b]thiophene, a gum, [468];

*cis*-6-chloro-2,3-dihydro-3-[4'-(5'-methyl-3'-phenylisoxazolyl)methoxy]-2-(1''-imidazolylmethyl)benzo[b]thiophene, m.p. 134—135°C.; and

*cis*-6-chloro-3-{8'-chloro-1',3'-dioxolo[4',5'-g]quinolin-7'-ylmethoxy}-2,3-dihydro-2-(1''-imidazolylmethyl)benzo[b]thiophene, m.p. 212—214°C.

### Example 12

*cis*-3-(3'-thenyloxy)-2,3-dihydro-2-(1'-imidazolylmethyl)benzo[b]thiophenes

A) In the procedure of Example 1A, substitute for 2-chloro-3-thenyl bromide an equivalent quantity of 3-thenyl bromide to obtain *cis*-6-chloro-3-(3'-thenyloxy)-2,3-dihydro-2-(1''-imidazolylmethyl)benzo[b]thiophene, m.p. 98—100°C.

B) In a similar manner, treat each of the *cis*-2,3-dihydro-3-hydroxy-2-(1'-imidazolylmethyl)benzo[b]thiophenes of Preparations 1B and 5 with 3-thenyl bromide to obtain, respectively:

a) *cis*-3-(3'-thenyloxy)-2,3-dihydro-2-(1''-imidazolylmethyl)benzo[b]thiophene,
b) *cis*-5-chloro-3-(3'-thenyloxy)-2,3-dihydro-2-(1''-imidazolylmethyl)benzo[b]thiophene,
c) *cis*-7-chloro-3-(3'-thenyloxy)-2,3-dihydro-2-(1''-imidazolylmethyl)benzo[b]thiophene,
d) *cis*-4,6-dichloro-3-(3'-thenyloxy)-2,3-dihydro-2-(1''-imidazolylmethyl)benzo[b]thiophene,
e) *cis*-5,6-dichloro-3-(3'-thenyloxy)-2,3-dihydro-2-(1''-imidazolylmethyl)benzo[b]thiophene,
f) *cis*-5,7-dichloro-3-(3'-thenyloxy)-2,3-dihydro-2-(1''-imidazolylmethyl)benzo[b]thiophene,
g) *cis*-6-trifluoromethyl-3-(3'-thenyloxy)-2,3-dihydro-2-(1''-imidazolylmethyl)benzo[b]thiophene,
h) *cis*-6-fluoro-3-(3'-thenyloxy)-2,3-dihydro-2-(1''-imidazolylmethyl)benzo[b]thiophene,
i) *cis*-7-fluoro-3-(3'-thenyloxy)-2,3-dihydro-2-(1''-imidazolylmethyl)benzo[b]thiophene.

*cis*-6-chloro-3-(3'-pyridylmethoxy)-2,3-dihydro-2-(1''-imidazolylmethyl)benzo[b]thiophene is prepared by the memthod of Example 2 from *cis*-6-chloro-2,3-dihydro-3-hydroxy-2-(1'-imidazolylmethyl)benzo[b]thiophene (4.00 g., 15.0 mmole), dry dimethylformamide (40 ml.), sodium hydride (50% dispersion, 1.51 g., 31.5 mmole) and 3-pyridylmethyl chloride hydrochloride (2.71 g., 16.5 mmole), except that 1% methanol in chloroform containing 0.2% (v/v) concentrated ammonium hydroxide is used as eluant.

Dissolve the free base (a gum) in a mixture of anhydrous ether (500 ml.) and 2-propanol (10 ml.). Add dropwise a saturated solution of HCl gas in 2-propanol until precipitation is complete. Filter the solution and dry the resulting residue. Redissolve the solid in water and lyophilize to give *cis*-6-chloro-3-(3'-pyridylmethoxy)-2,3-dihydro-2-(1''-imidazolylmethyl)benzo[b]thiophene dihydrochloride, [358].

### Example 14

5-chloro-3-(2'-chloro-3'-thenyloxy)-2,3-dihydro-2-(1''-imidazolylmethyl)benzo[b]furan is prepared by the method of Example 1A from 5-chloro-2,3-dihydro-3-hydroxy-2-(1'-imidazolylmethyl)benzo[b]furan (4.00 g., 8.3 mmole), dry dimethylformamide (40 ml.), sodium hydride (50% dispersion, 0.44 g., 9.1 mmole), and 2-chloro-3-thenyl bromide (1.93 g., 9.1 mmole).

### Example 15

*cis*-6-chloro-3-(2'-chloro-3'-thenyloxy-2,3-dihydro-2-(1''-imidazolylmethyl)benzo[b]thiophene hydrochloride

Dissolve *cis*-6-chloro-3-(2'-chloro-3'-thenyloxy)-2,3-dihydro-2-(1''-imidazolylmethyl)benzo[b]thiophene (2.0 g.) in anhydrous ether (400 ml.). Add dropwise a saturated solution of HCl gas in 2-propanol until precipitation is complete, filter, and dry the resulting residue to obtain the title compound.

The free base products of the preceding Examples, especially Examples 1B to 7 and 12 to 14, may be treated in a similar manner to yield the corresponding hydrochloride salts.

The following further compounds can be prepared by the methods of the foregoing Examples:

*cis*-5-t-butyl-3-(2'-chloro-3'-thenyloxy)-2,3-dihydro-2-(1''-imidazolylmethyl)benzo[b]thiophene, [417];

*cis*-5-t-butyl-3-(2'-chloro-3'-pyridylmethoxy)-2,3-dihydro-2-(1''-imidazolylmethyl)benzo[b]thiophene dihydrochloride, [434];

*cis*-5-t-butyl-3-{[5'-(4''-acetyl-1''-piperazinyl)-1',2',4'-thiadiazoyl-3']methoxy}-2,3-dihydro-2-(1'''-imidazolylmethyl)benzo[b]thiophene, [512];

*cis*-6-chloro-3-(2′-chloro-3′-pyridylmethoxy)-2,3-dihydro-2-(1″-imidazolylmethyl)benzo[b]thiophene, [391]

*cis*-6-fluoro-2,3-dihydro-2-(1″-imidazolylmethyl)-3-(3-thenyloxy)benzo[b]thiophene, [346];

5-chloro-3-(2′-chloro-3′-thenyloxy)-2-(1″-imidazolylmethyl)benzo[b]thiophene, m.p. 124°C.; and

6-chloro-3-(2′-chloro-3′-thenyloxy)-2-(1″-imidazolylmethyl)benzo[b]thiophene, m.p. 84°C.

The compounds of formula I, especially those of formula II, and their salts are valuable antifungal agents as demonstrated by *in vivo* tests in animals, e.g. a hamster *Candida* infection model, a guinea pig dermatophyte infection model and a mouse systemic *Candida* infection model. These tests indicate that the compounds of this invention are broad-spectrum antifungal agents active topically, orally and parenterally against topical dermatophyte and vaginal and systemic yeast infections.

In general, the dosage of compounds of formula I employed to combat a given fungal infection is similar to the dosage requirements of micronazole, clotrimazole, and ketoconazole, although the particular dosage level and the mode of administration will vary according to the particular host and the type and severity of the infection.

The compounds of this invention also have agricultural and industrial use. In agriculture, the compounds may be applied directly to plants or soil. The carriers may be powders such as talc, mica or clay, or sprays such as aqueous solutions with or without a solid carrier and a wetting agent. Industrially, the compounds may be used to disinfect glassware, medical equipment, and the like, by rinsing, contacting or impregnating the infected surface with the compound in a suitable carrier. Additionally, the compounds may be used to prevent growth of fungi in plants and other industrial materials susceptible to such growth.

The invention therefore provides compositions having antimicrobial activity, comprising a compound of formula I or acid addition salt thereof together with a carrier. The carrier may for example be an industrial material (such as a paint) which it is desired to preserve from microbial contamination; under these circumstances, the acid addition salt need not necessarily be pharmaceutically acceptable, and indeed anions of acids having antimicrobial activity may if desired, be used to enhance or extend the spectrum of antimicrobial activity of the compounds of formula I. However, the acid addition salt will normally be pharmaceutically acceptable, especially when the compounds are to be used as medicines.

Pharmaceutical formulations will comprise an antifungally, antibacterially or antiprotozoally effective amount of a compound of formula I or pharmaceutically acceptable acid addition salt thereof together with a pharmaceutically acceptable nontoxic carrier or excipient, especially for topical, oral or parenteral administration.

Topical pharmaceutical dosage forms may be prepared according to procedures well known in the art, and may contain a variety of ingredients as carriers. Formulations for topical use include ointments, creams, lotions, powders, aerosols and sprays. Ointments, lotions, and creams may contain water, oils, fats, waxes, polyesters, alcohols, or polyols, and fragrances, emulsifiers and preservatives. Powders are made by mixing the active ingredient with a readily available, inert, pulverous distributing agent, such as talcum, calcium carbonate, tricalcium phosphate, or boric acid. Aqueous suspensions of the above powders may also be made. Solutions or emulsions may be prepared using inert solvents which are preferably nonflammable, odorless, colorless, and non-toxic, for example vegetable oils, isopropanol, dimethyl sulfoxide, hydrogenated naphthalenes and alkylated naphthalenes. Similarly, aerosol or non-aerosol sprays may be prepared using solutions or suspensions in appropriate solvents, e.g. difluorodichloromethane for aerosols.

Topical formulations, e.g. ointments, creams, lotions, powders, or sprays, will usually contain about 0.1 to 3 grams of compound of formula I per 100 grams of carrier. Dosage units conveniently contain from 2 to 250, preferably 5 to 125 mg. of active ingredients per dosage unit.

Oral forms include elixirs, suspensions, and dosage units such as tablets and capsules. Tablets contain such excipients as starch or lactose; liquid forms may contain coloring or flavoring agents.

Parenteral forms to be injected intravenously, intramuscularly or subcutaneously are usually in the form of a sterile solution, and may contain salts or glucose to make the solution isotonic.

The following formulations illustrate compositions containing an antimicrobial agent according to the invention. The active ingredient is designated by the term "Drug", which is meant to indicate a compound according to the invention, especially one of the following compounds:

*cis*-6-fluoro-3-(2′-chloro-3′-thenyloxy)-2,3-dihydro-2-(1″-imidazolylmethyl)benzo[b]thiophene;

*cis*-5-fluoro-3-(2′-chloro-3′-thenyloxy)-2,3-dihydro-2-(1″-imidazolylmethyl)benzo[b]thiophene;

*cis*-6-chloro-3-(2′-chloro-3′-thenyloxy)-2,3-dihydro-2-(1″-imidazolylmethyl)benzo[b]thiophene; and

*cis*-6-chloro-3-(3′-thenyloxy)-2,3-dihydro-2-(1″-imidazolylmethyl)benzo[b]thiophene.

The formulations are prepared by standard methods.

Formulation I

| Tablet A | 7.50 mg. tablet |
|---|---|
| Drug | 7.50 mg. |
| Lactose, Anhydrous | 114.14 mg. |
| Starch (Sta-Rx 1500) | 54.66 mg. |
| Sodium lauryl sulfate | 5.00 mg. |
| Microcrystalline cellulose | 16.44 mg. |
| Silica gel | 0.41 mg. |
| Magnesium stearate | 1.85 mg. |
| Water (evaporates) | (0.05) mg. |

Formulation II

| Tablet B | 125.00 mg. tablet |
|---|---|
| Drug | 125.00 mg. |
| Polyethylene glycol 6000 | 100.00 mg. |
| Sodium lauryl sulfate | 6.25 mg. |
| Corn starch | 30.0 mg. |
| Lactose, anhydrous | 87.25 mg. |
| Magnesium stearate | 1.50 mg. |

Formulations III and IV

| | III | IV |
|---|---|---|
| *Capsule* | *25 mg. capsule* | *5.00 mg. capsule* |
| Drug | 25.00 mg. | 5.00 mg. |
| Microcrystalline cellulose | 312.00 mg. | — |
| Sodium lauryl sulfate | 5.00 mg. | 25.00 mg. |
| Corn start | 90.00 mg. | 310.00 mg. |
| Hydroxypropyl methylcellulose | 13.50 mg. | — |
| Magnesium stearate | 4.50 mg. | — |
| Water (evaporates) | | — |

Formulation V

| Injection | mg./ml. |
|---|---|
| Drug | 10.00 |
| Methyl *p*-hydroxybenozate | 1.30 |
| Propyl *p*-hydroxybenzoate | 0.20 |
| Sodium bisulfite | 3.20 |
| Disodium edetate | 0.10 |
| Sodium sulfate | 2.60 |
| Water from injection q.s. ad | 1.0 ml. |

Formulation VI

| Injectable Suspension | mg./ml. |
|---|---|
| Drug | 10.0 |
| Benzyl alcohol | 9.0 |
| Methyl *p*-hydroxybenzoate | 1.8 |
| Propyl *p*-hydroxybenozate | 0.2 |
| Sodium carboxymethylcellulose | 5.0 |
| Polyethylene glycol 4000 | 10.0 |
| Povidone | 5.0 |
| Sodium citrate | 15.0 |
| Disodium edetate | 0.1 |
| Water for injection q.s. ad | 1.0 ml. |

Formulation VII

| Sterile Powder for Injection | |
|---|---|
| Drug (sterile powder) | 20 mg. |
| Water for injection* | 10—200 ml. |

*add sterile or bacteriostatic water for injection, USP water for reconstruction.

Formulation VIII

| Cream | A | B | C |
|---|---|---|---|
| Drug | 0.5 mg. | 5.0 mg. | 10.0 mg. |
| Sorbitan monostearate | 20.0 | 20.0 | 20.0 |
| Polysorbate 60 | 15.0 | 15.0 | 15.0 |
| Spermaceti synthetic | 30.0 | 30.0 | 30.0 |
| Cetostearyl alcohol | 100.0 | 100.0 | 100.0 |
| Octyl dodecanol | 135.0 | 135.0 | 135.0 |
| Benzyl alcohol | 10.0 | 10.0 | 10.0 |
| Purified water to make 1 g. | | | |

Formulation IX

| Gel | A | B | C |
|---|---|---|---|
| Drug | 0.5 mg. | 5.0 mg. | 10.0 mg. |
| Butylated hydroxytoluene | 5.0 | 5.0 | 5.0 |
| Carbomer 940 | 15.0 | 15.0 | 15.0 |
| Propylene gylcol | 300.0 | 300.0 | 300.0 |
| Sodium hydroxide | 0.6 | 0.6 | 0.6 |
| Polyethylene glycol 400 to make 1 g. | | | |

*Formulation X*

| *Ointment* | *A* | *B* | *C* |
|---|---|---|---|
| Drug | 0.5 mg. | 5.0 mg. | 10.0 mg. |
| Mineral oil | 50.0 | 50.0 | 50.0 |
| White petrolatum to make 1 g. | | | |

Formulation XI

| *Lotion* | *A* | *B* | *C* |
|---|---|---|---|
| Drug | 0.5 mg. | 5.0 mg. | 10.0 mg. |
| Sorbitan monostearate | 2.0 | 2.0 | 2.0 |
| Polysorbate 60 | 30.0 | 30.0 | 30.0 |
| Cetyl esters wax | 30.0 | 30.0 | 30.0 |
| Cetostearyl alcohol | 40.0 | 40.0 | 40.0 |
| Octyl dodecanol | 40.0 | 40.0 | 40.0 |
| Benzyl alcohol | 20.0 | 20.0 | 20.0 |
| Purified water to make 1 gram | | | |

Formulation XII

| Spray Solution | A | B | C |
|---|---|---|---|
| Drug | 0.5 mg. | 5.0 mg. | 10.0 mg. |
| Propylene glycol | 200.0 | 200.0 | 200.0 |
| Lanoxol AWS | 5.0 | 5.0 | 5.0 |
| Alcohol | 300.0 | 300.0 | 300.0 |

Purified water to make 1 gram

Formulation XIII

| Powder | A | B | C |
|---|---|---|---|
| Drug | 0.5 | 5.0 | 10.0 |
| Starch | 100.0 | 100.0 | 100.0 |

Talc to make 1 g.

In the following tables, which give test results for a compound of formula I and for a number of representative known antimicrobials, the test compound according to the invention (designated 'Compound A' below) is *cis*-6-chloro-3-(2'-chloro-3'-thenyloxy)-2,3-dihydro-2-(1''-imidazolylmethyl)benzo-[b]thiophene.

TABLE 1

This Table gives, for various antimicrobials, the concentration (in ppm) that is 100% cidal against *Trichomonas vaginalis:*

| Ketoconazole | Miconazole | Clotrimazole | Compound A |
|---|---|---|---|
| 160 | 80 | 160 | 20 |

TABLE 2

This Table gives, for various antimicrobials, the $LD_{50}$ in mice *i.v.* in mg./kg.:

| Tioconazole | Ketoconazole | Miconazole | Compound A |
|---|---|---|---|
| 60—80 | 90—110 | 90—110 | 120—140 |

**Claims for Contracting States: BE CH DE FR GB IT LI NL SE**

1. A compound of the formula

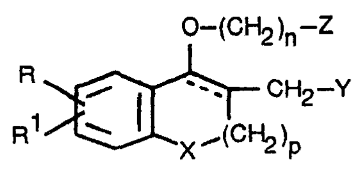

I

wherein

*n* is 0 or an integer from 1 to 4;

*p* is 0 or 1;

R and $R^1$ are independently hydrogen or halogen atoms, or lower alkyl, halolower alkyl, nitro or $NR^2R^3$ groups, wherein $R^2$ and $R^3$ are independently hydrogen atoms or lower alkyl groups; or R and $R^1$ together in adjacent positions complete a fused saturated or unsaturated carbocyclic ring that may contain 5 to 7 carbon atoms;

X is an oxygen or sulfur atom or a sulphinyl or sulphony group;

Y is an imidazolyl or 1,2,4-triazolyl group, either of which may be substituted by a lower alkyl group or by a phenyl, halophenyl or lower alkyl phenyl group;

Z is an alkyl group having 1 to 10 carbon atoms, an alkenyl or alkynyl group having 3 to 10 carbon atoms, a cycloalkyl group having 3 to 6 carbon atoms or a heterocyclic aromatic group selected from imidazolyl, thiazolyl, isothiazolyl, pyrazolyl, pyridyl, thienyl, thiadiazolyl, quinonlyl and furanyl, said heterocyclic aromatic groups being unsubstituted or substituted by at least one substituent selected from halogen atoms and lower alkyl, lower alkoxy, lower alkylidenedioxy, phenyl and N-(N'-lower alkanoylpiperazinyl) groups, provided that, when *n* is 0, Z is not 1-alkynyl; and when *n* is 1 to 4, Z can also be an alkoxy or alkylthio group having 1 to 10 carbon atoms, or phenyl or phenoxy group, or a phenyl or phenoxy group having substituents selected from halogen atoms and lower alkyl, lower alkoxy, lower alkylidenedioxy and N-(N'-lower alkanoylpiperazinyl) groups;

where the term "lower" indicates groups having 1 to 6 carbon atoms;

and the broken line indicates a single or double bond, with the proviso that it is a double bond only when *p* is 0 and X is a sulfur atom;

and the acid addition salts thereof.

2. Compounds and salts as claimed in claim 1 in the *cis*-form and having the formula

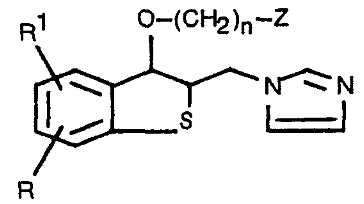

II

wherein

*n* is as defined in claim 1;

R and $R^1$ are independently hydrogen or halogen atoms, or lower alkyl, halolower alkyl, nitro or $NR^2R^3$ groups, wherein $R^2$ and $R^3$ are independently hydrogen atoms or lower alkyl groups;

Z is an alkyl, alkenyl, alkynyl, cycloalkyl or heterocyclic aromatic group defined in claim 1; provided that when *n* is 0, Z is not 1-alkynyl; and, when *n* is 1 to 4, Z can also be an alkoxy, alkylthio, phenyl, phenoxy or phenylthio group, or a phenyl group having substituents selected from halogen atoms and lower alkyl and N-(N'-lower alkanoylpiperazinyl) groups; the broken line indicates a single bond, and the term "lower" indicates groups having 1 to 4 carbon atoms.

3. Compounds and salts as claimed in claim 2 wherein

Z is a chlorophenyl, dichlorophenyl, thienyl, or 2-chlorothienyl group,

R and $R^1$ are hydrogen or halogen atoms, and

n is 1.

4. *cis*-6-Chloro-3-(2'-chloro-3'-thenyloxy)-2,3-dihydro-2-(1''-imidazolylmethyl)benzo[b]thiophene,
*cis*-6-chloro-3-(3'-thenyloxy-2,3-dihydro-2-(1''-imidazolylmethyl)benzo[b]thiophene,
*cis*-6-fluoro-3-(2'-chloro-3'-thenyloxy)-2,3-dihydro-2-(1''-imidazolylmethyl)benzo[b]thiophene,
*cis*-5-fluoro-3-(2'-chloro-3'-thenyloxy)-2,3-dihydro-2-(1''-imidazolylmethyl)benzo[b]thiophene,
*cis*-6-chloro-3-(2'-chloro-5'-thenyloxy)-2,3-dihydro-2-(1''-imidazolylmethyl)benzo[b]thiophene,

5. The pharmaceutically acceptable acid addition salts of the compounds of claims 1 to 4 with hydrochloric, sulfuric, phosphoric, hydrobromoic or nitric acid.

6. A process for the preparation of a compound of the formula I defined in claim 1 or an acid addition salt thereof, which comprises reacting a compound of the formula

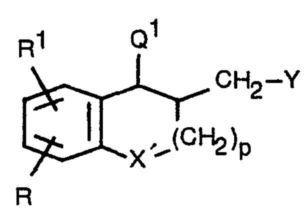

III

with a compound of the formula

$$Q^2—(CH_2)_n—Z \qquad IV$$

wherein R, $R^1$, Y, Z, *n* and *p* are as defined in claim 1, X' is an oxygen or sulfur atom, and one of $Q^1$ and $Q^2$ is a hydroxy group and the other is a reactive ester group, in the presence of an inert organic solvent and of an alkali metal base; whereafter, (1) for the preparation of a compound of the formula I wherein X is the group SO or $SO_2$ when X in the resulting product is S, said product is oxidised; and (ii) for the preparation

of a compound of the formula I wherein the broken line indicates a double bond, a resulting product wherein X is SO is dehydrated; and the compound of the formula I is isolated as the free base or as an acid addition salt thereof.

7. A process as claimed in claim 6 wherein the oxidation of a compound of formula I wherein X is sulfur to a compound of formula I wherein X is SO or $SO_2$ is effected with an organic peracid in an inert organic solvent.

8. A process as claimed in claim 6 or claim 7 wherein the dehydration of a compound of the formula I in which X is a group SO and the broken line indicates a single bond is effected by means of acetic anhydride or trifluoroacetic anhydride at elevated temperature in an inert organic solvent.

9. Compositions having antimicrobial activity, comprising a compound of formula I defined in claim 1 or acid addition salt thereof together with a carrier.

10. Compositions as claimed in claim 9 in the form of a pharmaceutical formulation comprising an antifungally, antibacterially or antiprotozoally effective amount of a compound of formula I or pharmaceutically acceptable acid addition salt thereof together with a pharmaceutically acceptable non-toxic carrier or excipient.

11. Compositions as claimed in claim 10 in a form adapted for topical, oral or parenteral administration and containing about 0.1 to 3 grams of compound of formula I or pharmaceutically acceptable acid addition salt thereof per 100 grams of carrier.

12. Compositions as claimed in claim 9 in the form of a dosage unit.

13. Compositions as claimed in claim 12 containing from 2 to 250 mg. of compound of formula I or pharmaceutically acceptable acid addition salt thereof per dosage unit.

14. Compositions as claimed in claim 9 in the form of agricultural compositions for application to plants or soil comprising an antimicrobially effective amount of a compound of formula I or acid addition salt thereof together with an agriculturally acceptable carrier; or in the form of industrial compositions comprising an industrial material susceptible to growth of fungi and an antifungally effective amount of a compound of formula I or acid addition salt thereof.

**Claims for the Contracting State: AT**

1. A process for the preparation of a compound of the formula

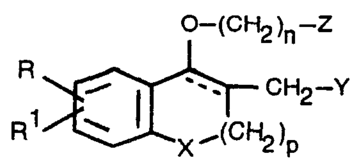

I

wherein

$n$ is 0 or an integer from 1 to 4;

$p$ is 0 or 1;

R and $R^2$ are independently hydrogen or halogen atoms, or lower alkyl, halolower alkyl, nitro or $NR^1R^3$ groups, wherein $R^2$ and $R^3$ are independently hydrogen atoms or lower alkyl groups; or R and $R^1$ together in adjacent positions complete a fused saturated or unsaturated carbocyclic ring that may contain 5 to 7 carbon atoms;

X is an oxygen or sulfur atom or a sulphinyl or sulphonyl group;

Y is an imidazolyl or 1,2,4-triazolyl group, either of which may be substituted by a lower alkyl group or by a phenyl, halophenyl or lower alkyl phenyl group;

Z is an alkyl group having 1 to 10 carbon atoms, an alkenyl or alkynyl group having 3 to 10 carbon atoms, a cycloalkyl group having 3 to 6 carbon atoms or a heterocyclic aromatic group selected from imidazolyl, thiazolyl, isothiazolyl, pyrazolyl, pyridyl, thienyl, thiadiazolyl, quinonlyl and furanyl, said heterocyclic aromatic groups being unsubstituted or substituted by at least one substituent selected from halogen atoms and lower alkyl, lower alkoxy, lower alkylidenedioxy, phenyl and N-(N'-lower alkanoylpiperazinyl) groups, provided that, when $n$ is 0, Z is not 1-alkynyl; and when $n$ is 1 to 4, Z can also be an alkoxy or alkylthio group having 1 to 10 carbon atoms, or phenyl or phenoxy group, or a phenyl or phenoxy group having substituents selected from halogen atoms and lower alkyl, lower alkoxy, lower alkylidenedioxy and N-(N'-lower alkanoylpiperazinyl) groups;

where the term "lower" indicates groups having 1 to 6 carbon atoms;

and the broken line indicates a single or double bond, with the proviso that it is a double bond only when $p$ is 0 and X is a sulfur atom;

or an acid addition salt thereof;

which comprises reacting a compound of the formula

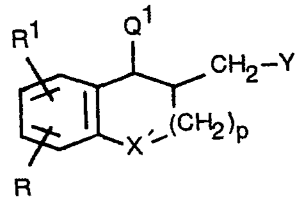

III

with a compound of the formula

$$Q^2\text{—}(CH_2)_n\text{—}Z \qquad IV$$

wherein R, $R^1$, Y, Z, *n*, *p* are as defined above, X' is an oxygen or sulfur atom, and one of $Q^1$ and $Q^2$ is a hydroxy group and the other is a reactive ester group, in the presence of an inert organic solvent and of an alkali metal base, whereafter, (i) for the preparation of a compound of the formula I wherein X is the group SO and $SO_2$ when X in the resulting product is S, said product is oxidised; and (ii) for the preparation of a compound of the formula I wherein the broken line indicates a double bond, a resulting product wherein X is SO is dehydrated; and the compound of the formula I is isolated as the free base or as an acid addition salt thereof.

2. A process as claimed in Claim 1 wherein
p is O;
*n* is as defined in claim 1;
X is S;
Y is 1-imidazolyl;
the broken line indicates a single bond;
R and $R^1$ are independently hydrogen or halogen atoms, or lower alkyl, halolower alkyl, nitro or $NR^2R^3$ groups, wherein $R^2$ and $R^3$ are independently hydrogen atoms or lower alkyl groups;
Z is an alkyl, alkenyl, alkynyl, cycloalkyl or heterocyclic aromatic group defined in claim 1; provided that when *n* is 0, Z is 1-alkynyl; and, when *n* is 1 to 4, Z can also be an alkoxy, alkylthio, phenyl or phenoxy group, or a phenyl group having substituents selected from halogen atoms and lower alkyl and N-(N'-lower alkanoylpiperazinyl) groups; the term "lower" indicates groups having 1 to 4 carbon atoms; and the compounds of formula I and III are in the *cis*-form.

3. A process as claimed in claim 2 wherein Z is a chlorophenyl, dichlorophenyl, thienyl or 2-chlorothienyl group, R and $R^1$ are hydrogen or halogen atoms, and *n* is 1.

4. A process as claimed in claim 3 wherein
R is a hydrogen atom; and
$R^1$ and Z are as follows

| $R^1$ | Z |
|---|---|
| 6-chloro | 2'-chloro-3'-thienyl |
| 6-chloro | 3'-thienyl |
| 6-fluoro | 2'-chloro-3'-thienyl |
| 5-fluoro | 2'-chloro-3'-thienyl |
| 6-chloro | 2'-chloro-5'-thienyl |

5. A process as claimed in any of claims 1 to 3 wherein the product of the formula I is isolated as a pharmaceutically acceptable acid addition salt with hydrochloric, sulfuric, phosphoric, hydrobromic or nitric acid.

6. A process as claimed in any of claims 1 to 5 wherein the reactive ester group represented by $Q^1$ or by $Q^2$ is a hydrocarbonsulfonyloxy group or a halogen atom, especially wherein $Q^2$ represents a chlorine or bromine atom.

7. A process as claimed in any of claims 1 to 6 wherein a compound of the formula III in which $Q^1$ is a hydroxy group is allowed to react with an alkali metal base in an inert anhydrous organic solvent and then the halide of the formula IV wherein $Q^2$ is a halogen atom is added thereto.

8. A process as claimed in claim 7 wherein the alkali metal base is an alkali metal hydride, an alkali metal hydroxide, an alkali metal amid or an alkali metal alcoholate and wherein the organic solvent is an amide, an aromatic hydrocarbon, an ether or a ketone.

9. A process as claimed in any of claims 1 to 8 wherein the oxidation of a compound of formula I wherein X is sulfur to a compound of formula I wherein X is SO or $SO_2$ is effected with an organic peracid in an inert organic solvent, or wherein the dehydration of a compound of the formula I in which X is a group

SO and the broken line indicates a single bond is effected by means of acetic anhydride or trifluoroacetic anhydride at elevated temperature in an inert organic solvent.

10. A process for the preparation of compositions having antimicrobial activity, which comprises admixing a compound of formula I defined in claim 1 or acid addition salt thereof together with a carrier.

11. A process as claimed in claim 10 for the preparation of a pharmaceutical formulation, which comprises admixing an antifungally, antibacterially or antiprotozoally effective amount of a compound of formula I or pharmaceutically acceptable acid addition salt thereof together with a pharmaceutically acceptable non-toxic carrier or excipient.

12. A process as claimed in claim 11 for the preparation of a composition in a form adapted for topical, oral or parenteral administration and containing about 0.1 to 3 grams of compound of formula I or pharmaceutically acceptable acid addition salt thereof per 100 grams of carrier.

13. A process as claimed in claim 10 for the preparation of a dosage unit.

14. A process as claimed in claim 13 for the preparation of dosage units containing from 2 to 250 mg. of compound of formula I or pharmaceutically acceptable acid addition salt thereof per dosage unit.

15. A process as claimed in claim 12 for the preparation of agricultural compositions for application to plants or soil, which comprises admixing an antimicrobially effective amount of a compound of formula I or acid addition salt thereof together with an agriculturally acceptable carrier; or for the preparation of industrial compositions, which comprises admixing or contacting an industrial material susceptible to growth of fungi and an antifungally effective amount of a compound of formula I or acid addition salt thereof.

**Patentansprüche für die Vertragsstaaten: BE CH DE FR BG IT LI NL SE**

1. Verbindung der Formel

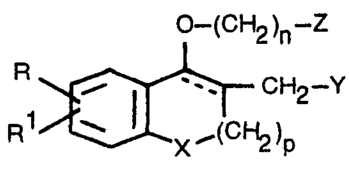

I

in der

n 0 oder eine ganze Zahl von 1 bis 4 ist,

p 0 oder 1 ist,

R und $R_1$ unabhängig voneinander Wasserstoff- oder Halogen-Atome oder Niederalkyl-, Halogenoniederalkyl-, Nitro- oder $NR^2R^3$-Gruppen sind, worin $R^2$ und $R^3$ unabhängig voneinander Wasserstoff-Atome oder Nidederalkyl-Gruppen sind, oder R und $R_1$ zusammen in benachbarten Positionen einen anellierten gesättigten oder ungesättigten carbocylischen Ring, der 5 bis 7 Kohlenstoff-Atome enthalten kann, vervollständigen,

X ein Sauerstoff- oder Schwefel-Atom oder eine Sulfinyl- oder Sulfonyl-Gruppe ist,

Y eine Imidazolyl- oder, 1,2,4-Triazolyl-Gruppe ist, die jeweils durch eine Niederalkyl-Gruppe oder durch eine Phenyl-, Halogenophenyl- oder Niederalkylphenyl-Gruppe, substituiert sein können,

Z eine Alkyl-Gruppe mit 1 bis 10 Kohlenstoff-Atomen, eine Alkenyl- oder Alkinyl-Gruppe mit 3 bis 10 Kohlenstoff-Atomen, eine Cycloalkyl-Gruppe mit 3 bis 6 Kohlenstoff-Atomen oder eine heterocyclische aromatische Gruppe ausgewählt aus Imidazolyl, Thiazolyl, Isothiazolyl, Pyrazolyl, Pyridyl, Thienyl, Thiadiazolyl, Chinolyl und Furanyl ist wobei; diese heterocyclischen aromatischen Gruppen unsubstituiert oder mit wenigstens einem Substituenten ausgewählt aus Halogen-Atomen und Niederalkyl-, Niederalkoxy-, Nideralkylidendioxy-, Phenyl- und N-(N'-Niederalkanoylpiperazinyl)-Gruppen haben, mit der Maßgabe, daß, wenn n 0 ist, Z nicht 1-Alkinyl ist, und wenn n 1 bis 4 ist, Z auch eine Alkoxy- oder Alkylthio-Gruppe mit 1 bis 10 Kohlenstoff-Atomen oder eine Phenyl- oder Phenoxy-Gruppe oder eine Phenyl- oder Phenoxy-Gruppe mit Substituenten ausgewählt aus Halogen-Atomen und Niederalkyl-, Niederalkoxy-, Niederalkylidendioxy-, Phenyl- und N-(N'-Niederalkanoylpiperazinyl)-Gruppen sein kann, wobei die Bezeichnung "Nieder" Gruppen mit 1 bis 6 Kohlenstoff-Atomen bezeichnet und die gestrichelte Linie eine Einfach- oder Doppelbindung bezeichnet, mit der Maßgabe, daß sie nur dann eine Doppelbindung bezeichnet, wenn p 0 ist und X ein Schwefel-Atom ist, und deren Säureadditionssalze.

2. Verbindungen und Salze nach Anspruch 1 in der *cis*-Form und mit der Formel

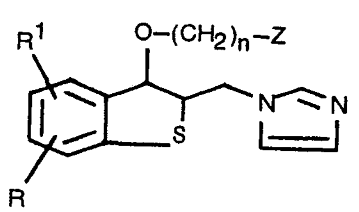

II

in der

n die in Anspruch 1 angegebene Bedeutung hat,

R und $R_1$ unabhängig voneinander Wasserstoff- oder Halogen-Atome oder Niederalkyl-, Halogen-oniederalkyl- Nitro- oder $NR^2R^3$-Gruppen sind, worin $R^2$ und $R^3$ unabhängig voneinander Wasserstoff-Atome oder Niederalkyl-Gruppen sind,

Z eine Alkyl-, Alkenyl-, Alkinyl-, Cycloalkyl- oder heterocyclische aromatische Gruppe wie in Anspruch 1 definiert ist, mit der Maßgabe, daß, wenn n 0 ist, Z nicht 1-Alkinyl ist, und wenn n 1 bis 4 ist, Z auch eine Alkoxy-, Alkylthio-, Phenyl-, Phenoxy, Phenyl, Phenoxy- oder Phenylthio-Gruppe oder eine Phenyl-Gruppe mit Substituenten ausgewählt aus Halogen-Atomen und Niederalkyl- und Niederalkanoylpiperazinyl)-Gruppen sein kann, wobei die gestrichelte Linie eine Einfachbindung bezeichnet und die Bezeichnung "Nieder" Gruppen mit 1 bis 4 Kohlenstoffen-Atomen bezeichnet.

3. Verbindung und Salze nach Anspruch 2, dadurch gekennzeichnet, daß

Z eine Chlorophenyl-, Dichlorophenyl-, Thienyl- oder 2-Chlorothienyl-Gruppe ist,

R und $R^1$ Wasserstoff- oder Halogen-Atome sind und n 1 ist.

4. *cis*-6-Chloro-3-(2'-chloro-3'-thenyloxy)-2,3-dihydro-2-(1''-imidazolylmethyl)benzo[b]thiophen,
*cis*-6-Chloro-3-(3'-thenyloxy)-2,3-dihydro-2-(1''-imidazolylmethyl)benzo[b]thiophen,
*cis*-6-Fluoro-3-(2'-chloro-3'-thenyloxy)-2,3-dihydro-2-(1''-imidazolylmethyl)benzo[b]thiophen,
*cis*-5-Fluoro-3-(2'-chloro-3'-thenyloxy)-2,3-dihydro-2-(1''-imidazolylmethyl)benzo[b]thiophen und
*cis*-6-Chloro-3-(2'-chloro-5'-thenyloxy)-2,3-dihydro-2-(1''-imidazolylmethyl)benzo[b]thiophen.

5. Pharmazeutisch annehmbare Säureadditionssalze der Verbindungen nach Anspruch 1 bis 4 mit Salzsäure, Schwefelsäure, Phosphorsäure, Bromwasserstoffsäure oder Salpetersäure.

6. Verfahren zur Herstellung einer Verbindung der Formel I nach Anspruch 1 oder eines Säureadditionssalzes derselben durch Reaktion einer Verbindung der Formel

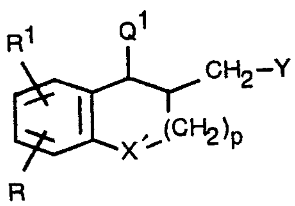

III

mit einer Verbindung der Formel

$$Q^2—(CH_2)_n—Z \quad IV$$

worin

R, $R^1$, Y, Z, n und p die in Anspruch 1 angegebenen Bedeutungen haben,

X' ein Sauerstoff- oder Schwefel-Atom is und eine der Gruppen

$Q^1$ und $Q^2$ eine Hydroxy-Gruppe und die andere eine reaktionsflähige Ester-Gruppe ist, in Gegenwart eines inerten organischen Lösungsmittels und einer Alkalimetall-Base, wonach

(i) zur Herstellung einer Verbindung der Formel I, in der X die Gruppe SO oder $So_2$ ist, sofern X in dem erhaltenen Produkt S ist, dieses Produkt oxidiert wird, und

(ii) zur Herstellung einer Verbindung der Formel I, in der die gestrichelte Linie eine Doppelbindung bezeichnet, ein erhaltenes Produkt, in dem X SO ist, dehydratisiert wird,

und die Verbindung der Formel I als freie Base oder als Säureadditionssalz derselben isoliert wird.

7. Verfahren nach Anspruch 6, dadurch gekennzeichnet, daß die Oxidation einer Verbindung der Formel I, in der X Schwefel ist, zu einer Verbindung der Formel I, in der X SO oder $SO_2$ ist, mit einer organischen Persäure in Gegenwart eines inerten organischen Lösungsmittels durchgeführt wird.

8. Verfahren nach Anspruch 6 oder Anspruch 7, dadurch gekennzeichnet, daß die Dehydratisierung einer Verbindung der Formel I, in der X eine Gruppe SO ist und die gestrichelte Linie eine Einfachbindung bezeichnet, mit Hilfe von Essigsäureanhydrid oder Trifluoroessigsäureanhydrid bei erhöhter Temperatur in einem inerten organischen Lösungsmittel durchgefühurt wird.

9. Zusammensetzungen mit antimikrobieller Aktivität, enthaltend eine Verbindung der Formel I nach Anspruch 1 oder deren Säureadditionssalz zusammen mit einem Träger.

10. Zusammensetzungen nach Anspruch 9 in Form einer pharmazeutischen Formulierung, die eine antifungal, antibakteriell oder antiprotozoal wirksame Menge einer Verbindung der Formel I nach Anspruch 1 oder eines Säureadditionssalzes derselben zusammen mit einem pharmazeutisch annehmbaren, nicht-toxischen Träger oder Streckmittel enthält.

11. Zusammensetzungen nach Anspruch 10 in einer für die topische, orale oder parenterale Verabreichung zubereiteten Form, die etwa 0, 1 bis 3 g der Verbindung der Formel I oder eines pharmazeutisch annehmbaren Säureadditionssalzes derselben auf 100 g Träger enthält.

12. Zusammensetzungen nach Anspruch 9 in Form einer Dosierungseinheit.

13. Zusammensetzungen nach Anspruch 12, enthaltend 2 bis 250 mg der Verbindung der Formel I oder eines pharmazeutisch annehmbaren Säureadditionssalzes derselben pro Dosierungseinheit.

14. Zusammensetzungen nach Anspruch 9 in Form landwirtschaftlich einsetzbarer Zusammensetzungen zur Anwendung auf Pflanzen oder Boden, enthaltend eine antimikrobiell wirksame Menge einer Verbindung der Formel I oder eines Säureadditionssalzes derselben zusammen mit einem landwirtschaftlich annehmbaren, Träger oder in Form industrieller Zusammensetzungen, enthaltend ein industrielles Material, das gegen Pilzbefall und -bewuchs empfindlich ist und eine antifugal wirksame Menge einer Verbindung der Formel I oder eines Säaureadditionssalzes derselben.

**Patentansprüche für den Vertragsstaat: AT**

1. Verfahren zur Herstellung einer Verbindung der Formel

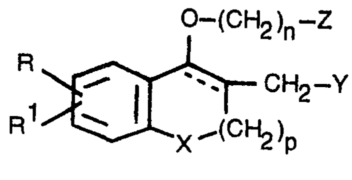

I

in der

n 0 oder eine ganze Zahl von 1 bis 4 ist,

p 0 oder 1 ist,

R und $R_1$ unabhängig voneinander Wasserstoff- oder Halogen-Atome oder Niederalkyl-, Halogenoniederalkyl-, Nitro- oder $NR^2R^3$-Gruppen sind, worin $n^2$ und $R^3$ unabhängig voneinander Wasserstoff-Atome oder Nidederalkyl-Gruppen sind, oder R und $R_1$ zusammen in benachbarten Positionen einen anellierten gesättigten oder ungesättigten carbocylischen Ring, der 5 bis 7 Kohlenstoff-Atome enthalten kann, vervollständigen,

X ein Sauerstoff- oder Schwefel-Atom oder eine Sulfinyl- oder Sulfonyl-Gruppe ist,

Y eine Imidazolyl- oder, 1,2,4-Triazolyl-Gruppe ist, die jeweils durch eine Niederalkyl-Gruppe oder durch eine Phenyl-, Halogenophenyl- oder Niederalkylphenyl-Gruppe, substituiert sein können,

Z eine Alkyl-Gruppe mit 1 bis 10 Kohlenstoff-Atomen, eine Alkenyl- oder Alkinyl-Gruppe mit 3 bis 10 Kohlenstoff-Atomen, eine Cycloalkyl-Gruppe mit 3 bis 6 Kohlenstoff-Atomen oder eine heterocyclische aromatische Gruppe ausgewählt aus Imidazolyl, Thiazolyl, Isothiazolyl, Pyrazolyl, Pyridyl, Thienyl, Thiadiazolyl, Chinolyl und Furanyl ist wobei diese heterocyclischen aromatischen Gruppen unsubstituiert oder mit wenigstens einem Substituenten ausgewählt aus Halogen-Atomen und Niederalkyl-, Niederalkoxy-, Niederalkylidendioxy-, Phenyl- und N-(N'-Niederalkanoylpiperazinyl)-Gruppen haben, mit der Maßgabe, daß, wenn n 0 ist, Z nicht 1-Alkinyl ist, und wenn n 1 bis 4 ist, Z auch eine Alkoxy- oder Alkylthio-Gruppe mit 1 bis 10 Kohlenstoff-Atomen oder eine Phenyl- oder Phenoxy-Gruppe oder eine Phenyl- oder Phenoxy-Gruppe mit Substituenten ausgewählt aus Halogen-Atomen und Niederalkyl-, Niederalkoxy-, Niederalkylidendioxy-, Phenyl- und N-(N'-Niederalkanoylpiperazinyl)-Gruppen sein kann, wobei die Bezeichnung "Nieder" Gruppen mit 1 bis 6 Kohlenstoff-Atomen bezeichnet und die gestrichelte Linie eine Einfach- oder Doppelbindung bezeichnet, mit der Maßgabe, daß sie nur dann eine Doppelbindung bezeichnet, wenn p 0 ist und X ein Schwefel-Atom ist, oder eines Säureadditionssalzes derselben, dadurch gekennzeichnet, daß eine Verbindung der Formel

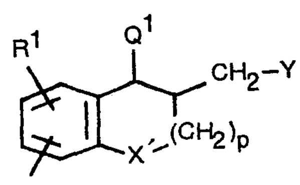

III

mit einer Verbindung der Formel

$$Q^2—(CH_2)_n—Z \quad IV,$$

worin R, $R^1$, Y, Z, n und p die im Vorstehenden angegebenen Bedeutungen haben,

X' ein Sauerstoff- oder Schwefel-Atom ist und eine der Gruppen

$Q^1$ und $Q^2$ eine Hydroxy-Gruppe und die andere eine reaktionsfähige Ester-Gruppe ist, in Gegenwart eines inerten organischen Lösungsmittels und einer Alkalimetall-Base umgesetzt wird, wonach

(i) zur Herstellung einer Verbindung der Formel I, in der X die Gruppe SO oder $SO_2$ ist, sofern X in dem erhaltenen Produkt S ist, dieses Produkt oxidiert wird, und

(ii) zur Herstellung einer Verbindung der Formel I, in der die gestrichelte Linie eine Doppelbindung bezeichnet, ein erhaltenes Produkt, in dem X SO ist, dehydratisiert wird,

und die Verbindung der Formel I als freie Base oder als Säureadditionssalz derselben isoliert wird.

2. Verfahren nach Anspruch 1 (Verfahren (a)), dadurch gekennzeichnet, daß
p 0 ist,
n die in Anspruch 1 angegebene Bedeutung hat,
X S ist,
Y 1-Imidazolyl ist, die gestrichelte Linie eine Eingach-Bindung bezeichnet,
R und $R_1$ unabhängig voneinander Wasserstoff- oder Halogen-Atome oder Niederalkyl-, Halogenoniederalkyl-, Nitro- oder $NR^2R^3$-Gruppen sind, worin $R^2$ und $R^3$ unabhängig voneinander Wasserstoff-Atome oder Niederalkyl-Gruppen, sind,
Z eine Alkyl-, Alkenyl-, Alkinyl-, Cycloalkyl- oder eine heterocyclische aromatische Gruppe wie in Anspruch 1 angegeben ist, mit der Maßgabe, daß,
wenn n 0 ist, Z nicht 1-Alkinyl ist, und
wenn n 1 bis 4 ist, Z auch eine Alkoxy-, Alkylthio-, Phenyl-, oder Phenoxy-Gruppe oder eine Phenyl-Gruppe mit Substituenten ausgewählt aus Halogen-Atomen und Niederalkyl- und N-(N'-Niederalkanoyl-piperazinyl)-Gruppen sein kann,
wobei die Bezeichnung "Nieder" Gruppen mit 1 bis 4 Kohlenstoff-Atomen bezeichnet und die Verbindungen der Formeln I und III in der *cis*-Form vorliegen.

3. Verfahren nach Anspruch 2, dadurch gekennzeichnet, daß
Z eine Chlorophenyl-, Dichlorophenyl-, Thienyl- oder 2-Chlorothienyl-Gruppe ist,
R und $R^1$ Wasserstoff- oder Halogen-Atome sind und
n 1 ist.

4. Verfahren nach Anspruch 3, dadurch gekennzeichnet, daß R ein Wasserstoff-Atom ist und $R^1$ und Z die nachstehenden Bedeutungen haben:

| R | Z |
|---|---|
| 6-Chloro | 2'-Chloro-3'-thienyl |
| 6-Chloro | 3'-Thienyl |
| 6-Fluoro | 2'-Chloro-3'-thienyl |
| 5-Fluoro | 2'-Chloro-3'-thienyl |
| 6-Chloro | 2'-Chloro-5'-thienyl. |

5. Verfahren nach irgendeinem der Ansprüche 1 bis 3, dadurch gekennzeichnet, daß das Produkt der Formel I als pharmazeutisch annehmbares Säureadditionssalz mit Salzsäure, Schwefelsäure, Phosphorsäure, Bromwasserstoffsäure oder Salpetersäure isoliert wird.

6. Verfahren nach irgendeinem der Ansprüche 1 bis 5, dadurch gekennzeichnet, daß die durch $Q^1$ oder durch $Q^2$ bezeichnete reaktionsfähige Ester-Gruppe eine Kohlenwasserstoffsulfonyloxy-Gruppe oder ein Halogen-Atom ist, insbesondere wo $Q^2$ ein Chlor- oder Brom-Atom bezeichnet.

7. Verfahren nach irgendeinem der Ansprüche 1 bis 6, dadurch gekennzeichnet, daß man eine Verbindung der Formel III, in der $Q^1$ eine Hydroxy-Gruppe ist, mit einer Alkalimetall-Base in einem inerten wasserfreien organischen Lösungsmittel reagieren läßt und anschließend das Halogenid der Formel IV, in der $Q^2$ ein Halogen-Atom ist, zugibt.

8. Verfahren nach Anspruch 7, dadurch gekennzeichnet, daß die Alkalimetall-Base ein Alkalimetall-Hydrid, ein Alkalimetall-Hydroxid, ein Alkalimetall-Amid oder ein Alkalimetall-Alkoholat ist und das organische Lösungsmittel ein Amid, ein aromatischer Kohlenwasserstoff, ein Ether oder ein Keton ist.

9. Verfahren nach irgendeinem der Ansprüche 1 bis 8, dadurch gekennzeichnet, daß die Oxidation einer Verbindung der Formel I, in der X Schwefel ist, zu einer Verbindung der Formel I, in der X SO oder $SO_2$ ist, mit einer organischen Persäure in Gegenwart eines inerten organischen Lösungsmittels durchgeführt wird, oder daß die Dehydratisierung einer Verbindung der Formel I, in der X eine Gruppe SO ist und die gestrichelte Linie eine Einfachbindung bezeichnet, mit Hilfe von Essigsäureanhydrid oder Trifluoroessigsäureanhydrid bei erhöhter Temperatur in einem inerten organischen Lösungsmittel durchgeführt wird.

10. Verfahren zur Herstellung von Zusammensetzungen mit antimikrobieller Aktivität, dadurch gekennzeichnet, daß eine Verbindung der Formel I nach Anspruch 1 oder deren Säureadditionssalz zusammen mit einem Träger vermischt wird.

11. Verfahren nach Anspruch 10 zur Herstellung von Zusammensetzungen in Form einer pharmazeutischen Formulierung, dadurch gekennzeichnet, daß eine antifungal, antibakteriell oder antiprotozoal wirksame Menge einer Verbindung der Formel I oder eines Säureadditionssalzes derselben zusammen mit einem pharmazeutisch annehmbaren, nicht-toxischen Träger oder Streckmittel vermischt wird.

12. Verfahren nach Anspruch 11 zur Herstellung von Zusammensetzungen in einer für die topische, orale oder parenterale Verabreichung zubereiteten Form, die etwa 0,1 bis 3 g der Verbindung der Formel I oder eines pharmazeutisch annehmbaren Säureadditionssalzes derselben auf 100 g Träger enthält.

13. Verfahren nach Anspruch 10 zur Herstellung von Zusammensetzungen in Form einer Dosierungseinheit.

14. Verfahren nach Anspruch 13 zur Herstellung von Dosierungseinheiten, die 2 bis 250 mg der Verbindung der Formel I oder eines pharmazeutisch annehmbaren Säureadditionssalzes derselben pro Dosierungseinheit enthalten.

15. Verfahren nach Anspruch 12 zur Herstellung von Zusammensetzungen in Form landwirtschaftlich einsetzbarer Zusammensetzungen zur Anwendung auf Pflanzen oder Boden, dadurch gekennzeichnet, daß eine antimikrobiell wirksame Menge einer Verbindung der Formel I oder eines Säureadditionssalzes derselben zusammen mit einem landwirtschaftlich annehmbaren Träger vermischt wird, oder in Form industrieller Zusammensetzungen, dadurch gekennzeichnet, daß ein industrielles Material, das gegen Pilzbefall und -bewuchs empfindlich ist und eine antifugal wirksame Menge einer Verbindung der Formel I oder eines Säureadditionssalzes derselben miteinander vermischt oder in Kontakt gebracht werden.

**Revendications pour les Etats contractants: BE CH DE FR GB IT LI NL SE**

1. Composé de formule

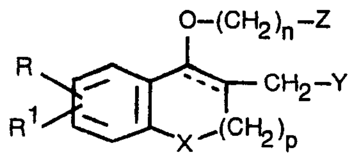

I

où *n* est 0 ou un nombre entier de 1 à 4;

*p* est 0 ou 1;

R et $R^1$ sont indépendamment des atomes d'hydrogène ou d'halogène, ou des groupes alcoyles inférieurs, haloalcoyles inférieurs, nitro ou $NR^2R^3$, où $R^2$ et $R^3$ sont indépendamment des atomes d'hydrogène ou de groupes alcoyles inférieurs;

ou bien R et $R^1$ ensemble à des positions adjacentes forment un noyau carbocyclique fusionné, saturé ou insaturé qui peut contenir 5 à 7 atomes de carbone;

X est un atome d'oxygène ou de soufre ou bien un groupe sulfinyle ou sulfonyle;

Y est un groupe imidazolyle ou 1,2,4-triazolyle, dont chacun peut être substitué par un groupe alcoyle inférieur ou par un groupe phényle, halophényle ou alcoyle inférieur phényle;

Z est un groupe alcoyle ayant 1 à 10 atomes de carbone, un groupe alkényle ou alkynyle ayant 3 à 10 atomes de carbone, un groupe cycloalcoyle ayant 3 à 6 atomes de carbone ou un groupe hétérocyclique aromatique choisi parmi les groupes imidazolyle, thiazolyle, isothiazolyle, pyrazolyle, pyridyle, thiényle, thiadiazolyle, quinoyle et furanyle, lesdits groupes aromatiques hétérocycliques étant non-substitués ou substitué par au moins un substituant choisi parmi des atomes d'halogène et des groupes alcoyles inférieurs, alcoxy inférieurs, alcoylidènedioxy inférieurs, phényle et N-(N'-alcanoyl inférieur pipérazinyle), à condition que, lorsque *n* est 0, Z ne soit pas 1-alkynyle; et lorsque *n* est 1 à 4, Z puisse également être un groupe alcoxy ou alkylthio ayant 1 à 10 atomes de carbone, ou un groupe phényle ou phénoxy, ou un groupe phényle ou phénoxy ayant des substituants choisis parmi des atomes d'halogène et des groupes alcoyles inférieurs, alcoxy inférieurs, alcoylidènedioxy inférieurs et N-(N'-alcanoyl inférieur-pipérazinyle);

où le terme "inférieur" indique des groupes ayant 1 à 6 atomes de carbone;

et la ligne en pointillé indique une simple ou double liaison à condition que ce soit une double liaison uniquement lorsque *p* est 0 et X est un atome de soufre;

et ses sels d'addition d'acide.

2. Composés et sels selon la revendication 1 sous la forme *cis-* et ayant la formule

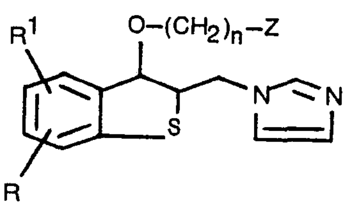

II

où *n* est tel que défini à la revendication 1;

R et $R^1$ sont indépendamment des atomes d'hydrogène ou d'halogène ou des groupes alcoyles inférieurs, haloalcoyles inférieurs, nitro ou $NR^2R^3$, où $R^2$ et $R^3$ sont indépendamment des atomes d'hydrogène ou des groupes alcoyles inférieurs;

Z est un groupe alcoyle, alkényle, alkynyle, cycloalcoyle ou hétérocyclique aromatique défini à la revendication 1; à condition que, lorsque *n* est O, Z ne soit pas 1-alkynyl; et lorsque *n* est 1 à 4, Z puisse également être un groupe alcoxy, alkylthio, phényle, phénoxy ou phénylthio ou un groupe phényle ayant

des substituants choisis parmi des atomes d'halogène et des groupes alcoyles inférieurs et N-(N'-alcanoyl inférieur pipérazinyle);
la ligne en pointillé indique une simple liaison, et le terme "inférieur" indique des groupes ayant 1 à 4 atomes de carbone.

3. Composés et sels selon la revendication 2 où Z est un groupe chlorophényle, dichlorophényle, thiényle ou 2-chlorothiényle.

R et $R^1$ sont des atomes d'hydrogène ou d'halogène, et *n* est 1.

4. *cis*-6-chloro-3-(2'-chloro-3'-thiényloxy)-2,3-dihydro-2-(1''-imidazolylmethyl)benzo[b]thiophène,
*cis*-6-chloro-3-(3'-thényloxy)-2,3-dihydro-2-(1''-imidazolylméthyl)benzo[b]thiophène,
*cis*-6-fluoro-3-(2'-chloro-3'-thényloxy)-2,3-dihydro-2-(1''-imidazolylméthyl)benzo[b]thiophène,
*cis*-5-fluoro-3-(2'-chloro-3'-thényloxy)-2,3-dihydro-2-(1''-imidazolylméthyl)benzo[b]thiophène, et
*cis*-6-chloro-3-(2'-chloro-5'-thényloxy)-2,3-dihydro-2-(1''-imidazolylméthyl)benzo[b]thiophène.

5. Les sels d'addition d'acide acceptables en pharmacie des composés des revendications 1 à 4 avec l'acide chlorhydrique, sulfurique, phosphorique, bromhydrique ou nitrique.

6. Procédé pour la préparation d'un composé de formule I défini à la revendication 1 ou son sel d'addition d'acide qui consiste à faire réagir un composé de formule

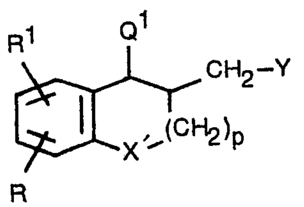

III

avec un composé de formule

$$Q^2-(CH_2)_n-Z \qquad IV$$

où R, $R^1$, Y, Z, *n* et *p* sont tels que définis à la revendication 1, X' est un atome d'oxygène ou de soufre, et l'un de $Q^1$ et $Q^2$ est un groupe hydroxy et l'autre est un groupe ester réactif,
en présence d'un solvant organique inerte et d'une base d'un métal alcalin;
ensuite (1) pour la préparation d'un composé de formule I où X est le groupe SO ou $SO_2$ lorsque X dans le produit résultant est S, ledit produit est oxydé;
et (ii) pour la préparation d'un composé de formule I où la ligne en pointillé indique une double liaison, un produit résultant où X est SO est déshydraté;
et le composé de formule I est isolé sous forme d'une base libre ou sous la forme de son sel d'addition d'acide.

7. Procédé selon la revendication 6 où l'oxydation d'un composé de formule I où X est du soufre en un composé de formule I où X est SO ou $SO_2$ est effectuée avec un peracide organique dans un solvant organique inerte.

8. Procédé selon la revendication 6 ou la revendication 7 où la déshydratation d'un composé de formule I où X est un groupe SO et où la ligne en pointillé indique une simple liaison est effectuée au moyen d'anhydride acétique ou d'anhydride trifluoroacétique à une température élevée dans un solvant organique inerte.

9. Compositions ayant une activité antimicrobienne, comprenant un composé de la formule I définie à la revendication 1 ou son sel d'addition d'acide avec un véhicule.

10. Compositions selon la revendication 9 sous la forme d'une formule pharmaceutique comprenant une quantité fongicidement, bactéricidement ou antiprotozoairement efficace d'un composé de formule I ou son sel d'addition d'acide acceptable en pharmacie avec un véhicule ou excipient non toxique pharmaceutiquement acceptable.

11. Compositions selon la revendication 10 sous une forme adaptée pour une administration topique, orale ou parentérale et contenant environ 0,1 à 3 grammes d'un composé de formule I ou son sel d'addition d'acide acceptable en pharmacie pour 100 grammes du véhicule.

12. Compositions selon la revendication 9 sous la forme d'une dose unitaire.

13. Compositions selon la revendication 12 contenant de 2 à 250 mg du composé de formule I ou son sel d'addition d'acide acceptable en pharmacie par dose unitaire.

14. Compositions selon la revendication 9 sous la forme de compositions pour l'agriculture, pour application à des plantes ou au sol, comprenant une quantité microbicidement efficace d'un composé de formule I ou son sel d'addition d'acide avec un véhicule acceptable en agriculture; ou sous la forme de compositions industrielles comprenant une matière industrielle sensible à la croissance des champignons et une quantité fongicidement efficace d'un composé de formule I ou son sel d'addition d'acide.

**Revendications pour l'Etat contractant: AT**

1. Un procédé pour la préparation d'un composé de formule

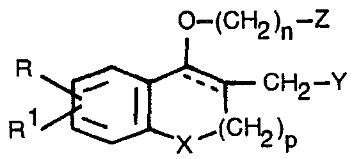

I

où *n* est 0 ou un nombre entier de 1 à 4;

*p* est 0 ou 1;

R et $R^1$ sont indépendamment des atomes d'hydrogène ou d'halogène, ou des groupes alcoyles inférieurs, haloalcoyles inférieurs, nitro ou $NR^2R^3$, où $R^2$ et $R^3$ sont indépendamment des atomes d'hydrogène ou de groupes alcoyles inférieurs;

ou bien R et $R^1$ ensemble à des positions adjacentes forment un noyau carbocyclique fusionné, saturé ou insaturé qui peut contenir 5 à 7 atomes de carbone;

X est un atome d'oxygène ou de soufre ou bien un groupe sulfinyle ou sulfonyle;

Y est un groupe imidazolyle ou 1,2,4-triazolyle, dont chacun peut être substitué par un groupe alcoyle inférieur ou par un groupe phényle, halophényle ou alcoyle inférieur-phényle;

Z est un groupe alcoyle ayant 1 à 10 atomes de carbone, un groupe alkényle ou alkynyle ayant 3 à 10 atomes de carbone, un groupe cycloalcoyle ayant 3 à 6 atomes de carbone ou un groupe hétérocyclique aromatique choisi parmi les groupes imidazolyle, thiazolyle, isothiazolyle, pyrazolyle, pyridyle, thiényle, thiadiazolyle, quinolyle et furanyle, lesdits groupes aromatiques hétérocycliques étant non-substitués ou substitué par au moins un substituant choisi parmi des atomes d'halogène et des groupes alcoyles inférieurs, alcoxy inférieurs, alcoylidènedioxy inférieurs, phényle et N-(N'-alcanoyl inférieur pipérazinyle), à condition que, lorsque *n* est 0, Z ne soit pas 1-alkynyle; et lorsque *n* est 1 à 4, Z puisse également être un groupe alcoxy ou alkylthio ayant 1 à 10 atomes de carbone, ou un groupe phényle ou phénoxy, ou un groupe phényle ou phénoxy ayant des substituants choisis parmi des atomes d'halogène et des groupes alcoyles inférieurs, alcoxy inférieurs, alcoylidènedioxy inférieurs et N-(N'-alcanoyl inférieur-pipérazinyle);

où le terme "inférieur" indique des groupes ayant 1 à 6 atomes de carbone;

et la ligne en pointillé indique une simple ou double liaison à condition que ce soit une double liaison uniquement lorsque *p* est 0 et X est un atome de soufre;

ou un sel d'addition d'acide;

qui comprend la réaction d'une composé de formule

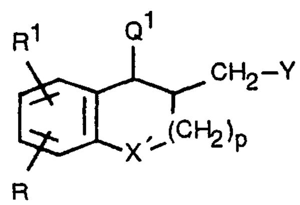

III

avec un composé de formule

$$Q^2—(CH_2)_n—Z \qquad IV$$

où R, $R^1$, Y, Z, *n* et *p* sont tels que définis ci-dessus,

X' est un atome d'oxygène ou de soufre,

et l'un de $Q^1$ et $Q^2$ est un groupe hydroxy et l'autre est un groupe ester réactif,

en présence d'un solvant organique inerte et d'une base d'un métal alcalin,

ensuite (i) pour la préparation d'un composé de formule I où X est le groupe SO ou $SO_2$ lorsque X dans le produit résultant est S, ledit produit est oxydé; et (ii) pour la préparation d'un composé de formule I où la ligne en pointillé indique une double liaison, un produit résultant où X est SO est déshydraté; et le composé de la formule I est isolé sous forme de base libre ou de son sel d'addition d'acide.

2. Un procédé selon la revendication 1 où p est 0;

*n* est tel que défini à la revendication 1;

X est S;

Y est 1-imidazolyle;

la ligne en pointillé indique une simple liaison;

R et $R^1$ sont indépendamment des atomes d'hydrogène ou d'halogène, ou des groupes alcoyles inférieurs, haloalcoyles inférieueurs, nitro ou $NR^2R^3$, où $R^2$ et $R^3$ sont indépendamment des atomes d'hydrogène ou des groupes alcoyles inférieurs;

Z est un groupe alcoyle, alkényle, alkynyle, cycloalcoyle ou hétérocyclique aromatique défini à la revendication 1; à condition que, lorsque *n* est 0, Z ne soit pas 1-alkynyle; et lorsque *n* est 1 à 4, Z puisse également être un groupe alcoxy, alkylthio, phényle ou phénoxy, ou bien un groupe phényle ayant des substituants choisis parmi des atomes d'halogène et des groupes alcoyles inférieurs et N-(N'-alcanoyl inférieur pipérazinyle);
le terme "inférieur" indique des groupes ayant 1 à 4 atomes de carbone;
et les composés de formule I et III sont sous la forme *cis*.

3. Un procédé selon la revendication 2 où Z est un groupe chlorophényle, dichlorophényle, thiényle ou 2-chlorothiényle, R et $R_1$ sont des atomes d'hydrogéne ou d'halogéne, et *n* est 1.

4. Un procédé selon la revendication 3 où R est un atome d'hydrogène; et $R_1$ et Z sont comme suit:

| $R^1$ | Z |
|---|---|
| 6-chloro | 2'-chloro-3'-thiényl |
| 6-chloro | 3'-thiényl |
| 6-fluoro | 2'-chloro-3'-thiényl |
| 5-fluoro | 2'-chloro-3'-thiényl |
| 6-chloro | 2'-chloro-5'-thiényl |

5. Un procédé selon l'une des revendications 1 à 3 où le produit de la formule I est isolé sous la forme d'un sel d'addition d'acide acceptable en pharmacie avec l'acide chlorhydrique, sulfurique, phosphorique, bromhydrique ou nitrique.

6. Un procédé selon l'une quelconque des revendications 1 à 5 où le groupe ester réactif représenté par $Q^1$ ou par $Q^2$ est un groupe hydrocarbonsulfonyloxy ou un atome d'halogène, en particulier où $Q^2$ représente un atome de chlore ou de brome.

7. Un procédé selon l'une quelconque des revendications 1 à 6 où l'on permet à un composé de la formule III où $Q^1$ est un groupe hydroxy de réagir avec une base d'un métal alcalin dans un solvant organique inerte anhydre puis l'halogénure de formule IV où $Q^2$ est un atome d'halogène lui est ajouté.

8. Un procédé selon la revendication 7 où la base d'un métal alcalin est un hydrure d'un métal alcalin, un hydroxyde d'un métal alcalin, un amide d'un métal alcalin ou un alcoolate d'un métal alcalin et où le solvant organique est un amide, un hydrocarbure aromatique, un éther ou une cétone.

9. Un procédé selon l'une quelconque des revendications 1 à 8 où l'oxydation d'un composé de formule I où X est du soufre en un composé de formule I où X est SO ou $SO_2$ est effectuée avec un peracide organique dans un solvant organique inerte, ou bien où
la déshydratation d'un composé de formule I où X est un groupe SO et la ligne en pointillé indique une simple liaison est effectuée au moyen d'anhydride acétique ou d'anhydride trifluoroacétique à une température élevée dans un solvant organique inerte.

10. Un procédé pour la préparation de compositions ayant une activité entimicrobienne, qui consiste à mélanger un composé de formule I défini à la revendication 1 ou son sel d'addition d'acide avec un véhicule.

11. Un procédé selon la revendication 10 pour la préparation d'une formule pharmaceutique, qui consiste à mélanger une quantité fongicidement, bactéricidement ou antiprotozoairement efficace d'un composé de formule I ou son sel d'addition d'acide acceptable en pharmacie avec un véhicule ou excipient non toxique acceptable en pharmacie.

12. Un procédé selon la revendication 11 pour la préparation d'une composition sous une forme adaptée pour une administration topique, orale ou parentérale et contenant environ 0,1 à 3 grammes du composé de formule I ou son sel d'addition d'acide acceptable en pharmacie pour 100 grammes du véhicule.

13. Un procédé selon la revendication 10 pour la préparation d'une dose unitaire.

14. Un procédé selon la revendication 13 pour la préparation de doses unitaires contenant de 2 à 250 mg du composé de formule I ou son sel d'addition d'acide acceptable en pharmacie par dose unitaire.

15. Un procédé selon la revendication 12 pour la préparation de compositions pour l'agriculture, pour l'application aux plantes ou au sol, qui consiste à mélanger une quantité microbicidement efficace d'un composé de formule I ou son sel d'addition d'acide à un véhicule acceptable en agriculture;
ou pour la préparation de compositions industrielles, qui consiste à mélanger ou à mettre en contact une matière industrielle susceptible d'une croissance de champignons et une quantité fongicidement efficace d'un composé de formule I ou son sel d'addition d'acide.